(19) Europäisches Patentamt
European Patent Office
Office européen des brevets

(11) EP 2 523 002 A1

(12) **EUROPEAN PATENT APPLICATION**

(43) Date of publication:
**14.11.2012 Bulletin 2012/46**

(21) Application number: **11166113.8**

(22) Date of filing: **13.05.2011**

(51) Int Cl.:
*G01N 33/543* *(2006.01)* *A61K 48/00* *(2006.01)*
*C12N 15/87* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Universität Bayreuth**
**95440 Bayreuth (DE)**

(72) Inventors:
- **Schallon, Anja**
**95447, Bayreuth (DE)**
- **Jérôme, Valérie**
**95448, Bayreuth (DE)**
- **Freitag, Ruth**
**95445, Bayreuth (DE)**
- **Synatschke, Christopher**
**95444, Bayreuth (DE)**
- **Majewski, Alexander**
**95444, Bayreuth (DE)**
- **Schmalz, Holger**
**95448, Bayreuth (DE)**
- **Müller, Axel**
**65193, Wiesbaden (DE)**

(74) Representative: **Neuefeind, Regina**
**Maiwald Patentanwalts GmbH**
**Elisenhof**
**Elisenstrasse 3**
**80335 München (DE)**

(54) **Utilization of magnetic nanoparticles as intracellular pull-down system**

(57) The present invention relates to a method for obtaining intracellular molecules having specific affinity for a polyelectrolyte particle comprising a functionalized magnetic nanoparticle by transferring said polyelectrolyte particle into a eukaryotic cell comprising the intracellular molecule, wherein a conjugate is formed between the polyelectrolyte particle and the intracellular molecule. By recovering the conjugate from the eukaryotic cell and isolating the intracellular molecule from the conjugate, the intracellular molecule can be obtained and subsequently analyzed. The present invention further relates to the use of such a polyelectrolyte particle for identifying and/or isolating a subset of eukaryotic cells comprising the polyelectrolyte particle from a plurality of eukaryotic cells, and for isolating a complex formed between the polyelectrolyte particle and a polyanion from polyelectrolyte particles not in complex with the polyanion. Furthermore, the present invention relates to the use of an intracellular molecule for modifying the expression of a nucleic acid transfected into a eukaryotic cell by means of such a polyelectrolyte particle.

EP 2 523 002 A1

## Description

## SUBJECT OF THE INVENTION

The present invention relates to a method for obtaining intracellular molecules having specific affinity for a polyelectrolyte particle comprising a functionalized magnetic nanoparticle by transferring said polyelectrolyte particle into a eukaryotic cell comprising the intracellular molecule, wherein a conjugate is formed between the polyelectrolyte particle and the intracellular molecule. By recovering the conjugate from the eukaryotic cell and isolating the intracellular molecule from the conjugate, the intracellular molecule can be obtained and subsequently analyzed. The present invention further relates to the use of such a polyelectrolyte particle for identifying and/or isolating a subset of eukaryotic cells comprising the polyelectrolyte particle from a plurality of eukaryotic cells, and for isolating a complex formed between the polyelectrolyte particle and a polyanion from polyelectrolyte particles not in complex with the polyanion. Furthermore, the present invention relates to the use of an intracellular molecule for modifying the expression of a nucleic acid transfected into a eukaryotic cell by means of such a polyelectrolyte particle.

## BACKGROUND OF THE INVENTION

Transfection, a process for delivering an exogenous specific gene into cells, is an indispensable way for obtaining useful information for the treatment of diseases and the development of medicines, by analyzing the signal transduction in which the protein coded by the gene is involved. In such transfection, trials have been made to use non-viral efforts in order to avoid harmful effects upon the organism. However, it is known in the art that there are big barriers to the improvement of transfection efficiency with a non-viral vector, including the passage through thecytoplasma membrane, the escape from transport vehicles, the nuclear import, and the nucleic acid release together with the following transcription reaction (E. Marshall (1999), Science 286: 2244-2245; S. Hacein-Bey-Abina (2003), Science 302: 415-419; D.W. Pack et a. (2005), Nat. Rev. Drug Discov. 4: 581-593).

Since the uptake of nucleic acids by eukaryotic cells is central to molecular biology and the application of molecular biology to clinical contexts, it has been sought to improve the efficiency of transfection processes since the introduction of DEAE and calcium phosphate mediated transfection techniques. This has given rise to a large number of protocols for delivering nucleic acid to cells, including techniques such as delivery using cationic polymers, lipofection, cationic liposome delivery, electroporation, gene gun and microinjection . However, the precise mechanism of transfection techniques still remains to be established and is difficult to assess with conventional analytical methods.

To escort genes from a solution to the cell nucleus, gene-delivery vectors must navigate a series of obstacles, both extracellular and intracellular. Viruses have evolved functions to address each challenge. However, by contrast, synthetic vectors are generally unsatisfactory because they lack one or more of the necessary functions. Consideration of the important barriers to gene delivery, therefore, is necessary to understand both the limitations of conventional synthetic vectors (such as cationic polymers) and is important for the rational design of new non-viral vectors.

Polyplexes protect DNA by sterically blocking the access of nucleolytic enzymes. Unprotected plasmid DNA is degraded by DNase within minutes, whereas plasmid DNA in polyplexes is stable for hours. Gene-delivery vectors generally bind to and condense DNA into small, compact structures through electrostatic interactions between the negative phosphates along the DNA backbone and positive charges displayed on the vector material. The resulting particles are typically toroidal or spherical structures with diameters ranging from about 30 to several hundred nanometres. Efforts have been made to better understand polyplex formation, but improvements in the theoretical understanding of the process and physico-chemical characterization of the resulting complexes are needed.

Untargeted polyplexes typically bind electrostatically to the surface of cells and are internalized via endocytosis. Alternatively polyplexes derivatized with targeting ligands bind to specific cell-surface receptors, in which case they are often internalized by receptor-mediated endocytosis. In either case, the polyplexes become localized within endocytic vesicles, which represent a hostile environment. The first vesicle, termed the early endosome, fuses with sorting endosomes from which the internalized material can be transported back to the membrane and out of the cell by exocytosis. More generally, however, polyplexes are believed to be trafficked into late endosomes. Polyplexes can subsequently be trafficked into lysosomes, which contain various degradative enzymes. The unprotonated amines of polycations such as PEI with different pKa values confer a buffering effect over a wide range of pH, which buffering property gives the polycation an opportunity to escape from the endosome (the so-called "proton sponge effect"). It is believed that this may prevent DNA degradation in the endosomal compartment during the maturation of the endosome to lysosome, facilitating intracellular trafficking of DNA (A Akinc et al. (2005), J Gene Med 7: 657-663; T. Bieber et al. (2002), Journal of Controlled Release 82: 441-454).

Once released from endosomal compartments, polyplexes must move through the cytoplasm to the nucleus. However, the cytoplasm is concentrated with proteins (i.e., actin filament, intermediate filament, microtubules) and organelles, all of which can hinder polyplex movement. Studies of mobility in the cytosol showed that diffusion is size-dependent (K. Luby-Phelps (1987), Proc. Natl Acad. Sci. USA 84: 4910-4913) and that DNA larger than 3,000 base pairs in length is essentially immobile. One might expect relatively large polyplexes to be immobile as well. This fact, together with the known degradation of DNA in the cytosol due to the presence of cytosolic nucleases, presents a critical barrier to efficient gene delivery.

Because the genome and nuclear machinery are vital to the various functions of the cell, nature has isolated the nucleus behind a double-bilayer membrane with tightly regulated pores that allow import and export of a specific set of biomolecules. The nuclear pore complex (NPC), a 125 000 kDa assembly of at least 30 distinct proteins, allows the passage of small molecules (< 5000 Da), but proteins larger than 10-20 kDa require active transport via specific nuclear import proteins (for example, Importins). Viruses have evolved mechanisms to utilize the nuclear import machinery of the cell. However, cationic polymers generally do not have this capacity and might rely largely on nuclear membrane breakdown during cell division for nuclear entry. As such, transport across the nuclear membrane, especially for non-dividing cells, seems to be a great obstacle to non-viral gene-delivery vectors.

It is well known that many proteins are naturally targeted to the nucleus by the presence of nuclear localization signals (NLS), which are short cationic peptide sequences that are recognized by importins. Due to their positive surface charge, it is possible that polyplexes could mimic NLS to a limited extent, but if so they must be very inefficient because very few vectors typically reach the nucleus. Further, nucleotide sequences on the genes themselves could provide some nuclear targeting. In fact, the nuclear import of polyplex vectors is one of the most poorly characterized steps in the gene-delivery process.

Finally, just as incorporation into a polymer complex protects DNA from enzymatic degradation, complexation prevents binding of the proteins required for gene expression. A vector must therefore release its DNA at some point in the delivery process. Cationic polymers must clearly be designed to incorporate a mechanism for nonspecific or environmentally responsive release of the genes, ideally close to the nucleus.

There is an ongoing need in the art to identify the important barriers to gene delivery, to understand the limitations of conventional synthetic vectors (such as cationic polymers) and to design novel non-viral vectors which overcome these drawbacks. However, the precise mechanism of transfection pathways is difficult to assess with conventional analytical methods, such as proteomics or conventional pull-down assays.

A major challenge in modern biology is directed to the understanding of the expression, function, and regulation of the entire set of proteins encoded by an organism, a technical field commonly known as proteomics. However, as there is no possibility to amplify proteins, research in this field is generally rather tedious because even a cell extract of a relatively simple prokaryotic organism contains a multitude of proteins encompassing a huge range of concentrations. Therefore, such a task is beyond the capabilities of any current single analytical method. Although useful for many applications the common proteomics techniques have major drawbacks where intracellular mechanisms or intracellular interactions between a non-viral vector and intracellular molecules are to be investigated.

Pull-down assays are *in vitro* methods typically used to determine a physical interaction between two or more proteins. Pull-down assays are useful for both confirming the existence of a protein-protein interaction predicted by other research techniques (e.g., co-immunoprecipitation) and as an initial screening assay for identifying previously unknown protein-protein interactions. Pull-down assays are a form of affinity purification and are very similar to immunoprecipitation, except that a "bait" protein is used instead of an antibody. In a pull-down assay, a bait protein is tagged and captured on an immobilized affinity ligand specific for the tag, thereby generating a "secondary affinity support" for purifying other proteins that interact with the bait protein. The secondary affinity support of immobilized bait is then incubated with a protein source that contains putative "prey" proteins, such as a cell lysate. The source of prey protein at this step depends on whether the researcher is confirming a previously suspected protein-protein interaction or identifying an unknown interaction. The method of protein elution depends on the affinity ligand and ranges from using competitive analytes to low pH or reducing buffers. Besides investigating the interaction of two or more proteins, pull-down assays are a powerful tool to detect the activation status of specific proteins.

Such a pull-down assay may also be used as a valuable discovery tool. In particular, the research interest lies in discovering new proteins in the endogenous environment that interact with a given bait protein. The endogenous environment can entail a plethora of possible protein sources but is generally characterized as a complex protein mixture considered to be the native environment of the bait protein. Any cellular lysate in which the bait is normally expressed, or complex biological fluid (i.e. blood, intestinal secretions, etc.) where the bait would be functional, is an appropriate prey protein source for discovery studies.

Since pull-down assays are a powerful detection in molecular biology, a large number of pull-down assays were developed, including the utilization of magnetic beads in such assays. For instance, dynabeads are superparamagnetic, monosized and spherical polymer particles with a consistent, defined surface for the adsorption or coupling of various bioreactive molecules or cells. They were developed after John Ugelstad managed to create uniform polystyrene spherical beads (defined as microbeads) of exactly the same size, at the University of Trondheim, Norway in 1976. This discovery revolutionised the separation of many biological materials. The technology behind the beads, called Dynabeads, was licensed to Dyno Industries in 1980 and this magnetic separation technology has been used for the

isolation and manipulation of biological material, including cells, nucleic acids, proteins and pathogenic microorganisms.

However, there is still no possibility to determine a physical interaction between a non-viral vector and intracellular molecules inside the cell. Such a task is beyond the capabilities of any current single pull-down assay. Hence, there is a strong demand in the art for providing a detection tool that would allow the detection of intracellular interactions between a non-viral vector (in particular, a cationic polymer) and intracellular molecules (such as, e.g., proteins).

## OBJECT AND SUMMARY OF THE INVENTION

It is an object of the invention to provide a detection tool that allows the detection of intracellular interactions between a non-viral vector (in particular, a cationic polymer) and intracellular molecules (such as, e.g., proteins). One further object of the invention is to provide a method for obtaining an intracellular molecule having affinity, in particular specific affinity, for a polyelectrolyte particle comprising a functionalized magnetic nanoparticle. Another object of the invention is to provide a pull-down system that allows the detection of intracellular interactions between a non-viral vector (in particular, a cationic polymer) and intracellular molecules (such as, e.g., proteins). Another object of the invention is to provide a pull-out system that allows the detection of intracellular interactions between a non-viral vector (in particular, a cationic polymer) and intracellular molecules (such as, e.g., proteins).

One further object of the invention is to provide a method or a tool that allows the isolation of cationic polymers that are in complex with a nucleic acid (so-called polyplexes) from cationic polymers that are not in complex with a nucleic acid. One further object of the invention is to provide a method or a tool that allows the isolation of eukaryotic cells containing a polyplex from eukaryotic cells not containing the polyplex. Another object of the invention is to provide a method or a tool that allows the identification of eukaryotic cells containing a polyplex from eukaryotic cells not containing the polyplex.

One further object of the invention is to provide an improved transfection protocol which allows targeted delivery and uptake of non-viral vectors to specific cells at high efficiency. Another object of the invention is to provide a targeted delivery system which is sufficiently specific to allow targeting of the non-viral vector to a specific cell type, yet also sufficiently efficient to ensure that a substantial proportion of the targeted cells are transfected whilst maintaining cell viability.

These objectives as well as others which will become apparent from the ensuing description are attained by the subject matter of the independent claims. Some of the embodiments of the present invention are defined by the subject matter of the dependent claims.

In one embodiment, the present invention relates to an *in vitro* method for obtaining an intracellular molecule having specific affinity for a polyelectrolyte particle, wherein the polyelectrolyte particle comprises at least 15 polymer chains, which have a positive net charge and radiate outward from a functionalized magnetic nanoparticle so as to form the polyelectrolyte particle, wherein each polymer chain comprises at least 40 covalently linked monomer units on average, and wherein the method comprises:

(a) contacting said polyelectrolyte particle and a eukaryotic cell or a microbioreactor, comprising the intracellular molecule, under conditions for interaction of said polyelectrolyte particle with the intracellular molecule to form a conjugate;
(b) recovering the conjugate from the eukaryotic cell or the microbioreactor; and
(c) isolating the intracellular molecule from the conjugate.

According to an optional embodiment, the polymer chains are linear polymer chains and the polyelectrolyte particle does not comprise any branched or cross-linked polymer chains.

In a specific embodiment of the invention, the polymer chains are each formed at least in part by the polymerisation of ethylenically unsaturated monomers selected from the group consisting of dialkylamino alkyl(alk)acrylates, dialkylamino alkyl(alk)acrylamides, hydroxy alkyl(alk)acrylates, hydroxy alkyl(alk)acrylamides, alkyl(alk)acrylates and alkyl(alk)acrylamides.

In a further specific embodiment of the invention, the polymer chains are each formed at least in part by the polymerisation of ethylenically unsaturated monomers comprising monomers having the following general formula (I):

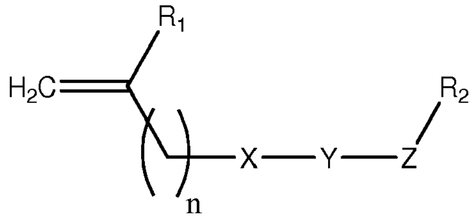

(I)

wherein:

$R_1$ is selected from hydrogen or optionally substituted $-(C_1-C_3)$alkyl;
X is selected from -O-C(O)-, -C(O)-O-, $-NT_3-C(O)-$ or $-C(O)-NT_3-$;
Y is $-(CH_2)_{m}-$;
Z is selected from -O-, $-(O-CH_2CH_2)_o-$, $-(CH_2CH_2-O)_o-$, -O-C(O)-, -C(O)-O-, $-NT_3-$, $- NT_3-C(O)-$ or $-C(O)-NT_3-$, or is absent;
n is an integer selected from 0, 1, or 2;
m is an integer selected from 0, 1, 2, 3, 4, or 5;
o is an integer selected from 1 to 10;
$R_2$ is selected from -H, or $-(C_1-C_3)$alkyl which is unsubstituted or substituted with 1 or 2 independently selected $R_4$ groups;
each $R_4$ is independently selected from $-OT_3$, $-N(T_1)(T_2)$, $-N(T_3)C(=O)T_3$, or $-N(T_3)C(=O)OT_3$; and

each $T_1$, $T_2$, and $T_3$ is independently selected from -H or $-(C_1-C_3)$alkyl.

In a further specific embodiment of the invention, the polymer chains are each formed at least in part by the polymerisation of ethylenically unsaturated monomers comprising monomers having a general formula selected from the group consisting of formulae (II), (III) and (IV), wherein formulae (II), (III) and (IV) have the following chemical structures:

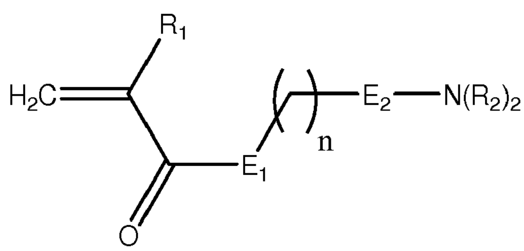

(II),

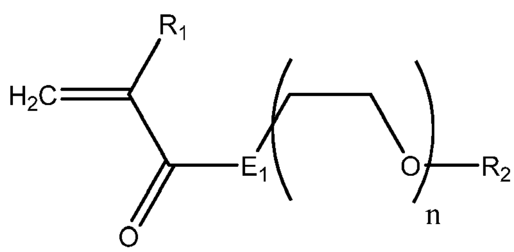

(III),

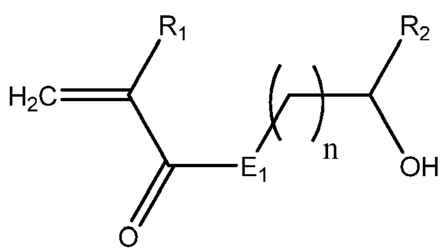

(IV),

wherein:

in formula (II):

$R_1$ is selected from -H, $-CH_3$ or $-CH_2CH_3$;
$E_1$ is selected from -O-, -NH-, or $-N(CH_3)-$;
n is an integer selected from 0, 1 or 2;
$E_2$ is selected from $-(NH-CH_2)_m-$, $-(CH_2-NH)_m-$, $-(NH-CH_2CH_2)_{m-}$, $-(CH_2CH_2-NH)_{m-}$, $-(N(CH_3)-CH_2)_m-$, $-(CH_2-N(CH_3))_m-$, $-(N(CH_3)-CH_2CH_2)_m$, $-(CH_2CH_2-N(CH_3))_m-$, or is absent;
m is an integer selected from 0, 1, 2, 3, 4, 5, or 6; and
$R_2$ is selected from -H, $-CH_3$ and $-CH_2CH_3$;

in formula (III):

$R_1$ is selected from -H, $-CH_3$ or $-CH_2CH_3$;
$E_1$ is selected from -O-, -NH-, or $-N(CH_3)-$;
n is an integer selected from 1, 2, 3, 4, 5, 6, 7, 8, 9, or 10; and
$R_2$ is selected from -H, $-CH_3$ and $-CH_2CH_3$;

in formula (IV):

$R_1$ is selected from -H, $-CH_3$ or $-CH_2CH_3$;
$E_1$ is selected from -O-, -NH-, or $-N(CH_3)-$;
n is an integer selected from 0, 1, 2, or 3; and
$R_2$ is selected from -H, $-CH_3$ and $-CH_2CH_3-$

Optionally, the functionalized magnetic nanoparticle is a functionalized maghemite nanoparticle, optionally a dopamine-functionalized maghemite nanoparticle based core.

Optionally, the polymer chains comprised by the polyelectrolyte particle together have a molecular weight of at least 500 kDa, 550 kDa, 600 kDa, 650 kDa, 700 kDa, 750 kDa, 800 kDa, 850 kDa, 900 kDa, 950 kDa, 1000 kDa, 1500 kDa, 2000 kDa, 2500 kDa, 3000 kDa, 3500 kDa, 4000 kDa, 4500 kDa, 5000 kDa, 6000 kDa, 7000 kDa, 8000 kDa, 9000 kDa, 10,000 kDa, 15,000 kDa, 20,000 kDa, or 30,000 kDa.

In a specific embodiment of the invention, the polyelectrolyte particle further comprises a polyanion selected from a nucleic acid or a protein. In a further specific embodiment of the invention, the polyelectrolyte particle further comprises a nucleic acid, optionally being selected from the group consisting of cDNA, genomic DNA, plasmid DNA, messenger RNA, antisense RNA, siRNA, short hairpin RNA, micro-RNA, ribozymes, transfer RNA, ribosomal RNA, and DNA encoding for such types of RNA.

In a specific embodiment of the invention, the intracellular molecule is selected from an RNA, genomic DNA, a lipid, a phospholipid, a carbohydrate, a peptide, an oligopeptide, a polypeptide, a monomeric protein and a multimeric protein.

According to a specific embodiment of the invention, the intracellular molecule comprises at least one functionality selected from the group consisting of:

(a) binding of the intracellular molecule to a ligand or antigen;
(b) exertion of a catalytic activity, optionally an enzymatic activity, by the intracellular molecule;
(c) susceptibility of the intracellular molecule to a catalytic activity, optionally an enzymatic activity;
(d) facilitation by the intracellular molecule of altered transport of the polyelectrolyte particle across a biological membrane;
(e) facilitation by the intracellular molecule of altered transport of the polyelectrolyte particle in the intracellular compartment;
(f) influence by the intracellular molecule on expression of at least one gene in a population of eukaryotic cells; and
(g) influence by the intracellular molecule on the growth or metabolism of a population of eukaryotic cells.

According to a very specific embodiment of the invention, the polyelectrolyte particle further comprises a nucleic acid coding for a target protein, and the method comprises:

(a) contacting said polyelectrolyte particle and a eukaryotic cell comprising the intracellular molecule under conditions for interaction of said polyelectrolyte particle with the intracellular molecule to form a conjugate;

(b) recovering the conjugate from the eukaryotic cell;
(c) isolating the intracellular molecule from the conjugate;
(d) optionally, characterizing and/or identifying the intracellular molecule;
(e) contacting the polyelectrolyte particle and a further eukaryotic cell under the conditions according to (a), wherein the further eukaryotic cell comprises a different amount of the intracellular molecule compared to the eukaryotic cell according to (a);
(f) comparing the amounts of target protein in the eukaryotic cell and in the further eukaryotic cell; and
(g) optionally, categorizing the intracellular molecule with regard to its capability to modify a transfection reaction.

Optionally, recovering the conjugate from the eukaryotic cell or the microbioreactor comprises

(a) releasing intracellular molecules from inside the eukaryotic cell by disruption of the eukaryotic cell, transferring the intracellular molecules on a column placed in a magnetic field and isolating the conjugate from the intracellular molecules; or
(b) releasing the conjugate from inside the eukaryotic cell or the microbioreactor by applying a magnetic field to the eukaryotic cell or the microbioreactor.

In a further embodiment, the present invention relates to the use of a polyelectrolyte particle as defined in any of claims 1 to 8 or as defined herein below for identifying and/or isolating a subset of eukaryotic cells comprising the polyelectrolyte particle from a plurality of eukaryotic cells, the use comprising contacting the polyelectrolyte particle and the plurality of eukaryotic cells under conditions to allow transfer of the polyelectrolyte particle into the eukaryotic cells, and identifying and/or isolating the subset of eukaryotic cells having magnetic properties.

In a further embodiment, the present invention relates to the use of a polyelectrolyte particle as defined in any of claims 1 to 8 or as defined herein below for isolating a complex formed between the polyelectrolyte particle and a nucleic acid from polyelectrolyte particles not in complex with the nucleic acid.

In a further embodiment, the present invention relates to the use of an intracellular molecule as obtained in the inventive method for modifying the expression of a nucleic acid transfected into a eukaryotic cell by means of a polyelectrolyte particle as defined in any of claims 1 to 8 or as defined herein below.

## FIGURE LEGENDS

The accompanying drawings, which are incorporated and form part of the specification, merely illustrate certain embodiments of the present invention. They are meant to serve to explain specific modes of the present invention to those of skilled in the art. In the drawings:

Figure 1 depicts a scheme of the synthesis strategy and dual-responsive behavior of the NP@PDMAEMA particles.

Figure 2A depicts the TEM-micrograph of oleic acid stabilized nanoparticles prepared by drop-coating from toluene ($c < 0.1$ g/L) on a copper grid. Figure 2B depicts the DLS curve of oleic acid stabilized nanoparticles ($c = 0.5$ g/L, $\Theta = 90^\circ$).

Figure 3A depicts the EDX spectrum of purified NP@Dopamine initiator. Figure 3B depicts the FT-IR spectra of pure dopamine based ATRP-initiator (a), pure oleic acid (b), purified nanoparticles covered by oleic acid NP@Oleic acid (c) and purified ATRP-initiator functionalized particles NP@Dopamine initiator (d).

Figure 4A depicts the TGA of pure dopamine based ATRP initiator. Figure 4B depicts the comparison of TGA traces of NP@Oleic acid (○) and NP@Dopamine initiator (■).

Figure 5 depicts a comparison of the TGA curves of a) NP@Dopamine initiator, b) NP@PDMAEMA$_{46}$ purified by precipitation and c) NP@PDMAEMA$_{160}$ purified by ultrafiltration.

Figure 6 depicts TEM micrographs of NP@PDMAEMA drop-coated from water onto hydrophylized copper grids at different magnifications.

Figure 7 depicts aqueous NP@PDMAEMA-solution under influence of a NdFeB-magnet below LCST (left) and above LCST (right).

Figure 8 depicts the normalized magnetization curves of NP@Oleic acid (●) and NP@PDMAEMA$_{160}$ (D), and

corresponding Ivanov fits (black line).

Figure 9A depicts turbidity measurements of $NP@PDMAEMA_{46}$ (c = 0.1 g/L) at different pH; $NP@PDMAEMA_{46}$ at pH 10 (■), $NP@PDMAEMA_{46}$ at pH 9 (Δ), $NP@PDMAEMA_{46}$ at pH 8 (●) and $NP@PDMAEMA_{46}$ at pH 7 (0).

Figure 9B depicts cloud points at 0.1 g/L of cleaved PDMAEMA (■), $NP@PDMAEMA_{46}$ (○) and dependent on the pH.

Figure 10 depicts the principle of the separation of complexes between $NP@PDMAEMA_{46}$-pDNA and intracellular macromolecules after transfection.

Figure 11 depicts the analysis of the isolated molecules via magnetic sorting. (1) Molecular weight markers in kDa; (2) cell lysate; (3) Isolated magnetic complexes $NP@PDMAEMA_{46}$-PMAA (complexe II); (4) Proteins that bound to the $NP@PDMAEMA_{46}$-pDNA conjugates I.

## DETAILED DESCRIPTION OF THE INVENTION

The present invention illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. Where the term "comprising" is used in the present description and claims, it does not exclude other elements or steps. For the purposes of the present invention, the term "consisting of" is considered to be a preferred embodiment of the term "comprising of'. If hereinafter a group is defined to comprise at least a certain number of embodiments, this is also to be understood to disclose a group which optionally consists only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of $\pm$ 10%, and optionally $\pm$ 5%.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used.

In one embodiment, the present invention relates to an *in vitro* method for obtaining an intracellular molecule having specific affinity for a polyelectrolyte particle, wherein the polyelectrolyte particle comprises at least 15 polymer chains, which have a positive net charge and radiate outward from a functionalized magnetic nanoparticle so as to form the polyelectrolyte particle, wherein each polymer chain comprises at least 40 covalently linked monomer units on average, and wherein the method comprises:

(a) contacting said polyelectrolyte particle and a eukaryotic cell or a microbioreactor, comprising the intracellular molecule, under conditions for interaction of said polyelectrolyte particle with the intracellular molecule to form a conjugate;
(b) recovering the conjugate from the eukaryotic cell or the microbioreactor; and
(c) isolating the intracellular molecule from the conjugate.

In particular, when used herein, the term "polyelectrolyte particle" denotes a particle that comprises a certain number of "polymer chains" (also designated as "polymer arms") radiating outward from a common branching centre so as to form the polyelectrolyte particle. Optionally, the polyelectrolyte particle is star-shaped. In the context of the present invention, the term "star-shaped particle" (as designated as "star polymer") concerns a macromolecule having a common branching centre (also designated as "central anchoring point" or "branch point") with branches radiating from the centre or point (i.e. the polymer arms). In a specific embodiment according to the present invention, the polymer chains of the polyelectrolyte particle possess a positive net charge at physiological pH. In the context of the present invention, the star-shaped polyelectrolyte particle is a so-called star polymer, i.e. a branched polymer molecule in which a single branch point gives rise to multiple linear chains or arms.

When used herein, the term "polymer" generally denotes a large molecule (macromolecule) composed of repeating structural units (i.e. monomer units), which subunits are typically connected by covalent chemical bonds. In

the context of the present invention, "polymerization" is the process of combining many small molecules known as monomers into a covalently bonded chain. During the polymerization process, some chemical groups may be lost from each monomer. The distinct piece of each monomer that is incorporated into the polymer is known as a "repeat unit", "monomer unit" or "monomer residue". The polymer nomenclature is generally based upon the type of monomer residues comprising the polymer. Polymers that contain only a single type of repeat unit are known as "homopolymers", while polymers containing a mixture of repeat units are known as "copolymers".

In the context of the present invention, a "polyelectrolyte particle" is a particle comprising several polymer arms whose repeating units bear electrolyte groups. For instance, these groups can accept protons in aqueous solutions (such as water), making the polymers charged. Polyelectrolyte properties are thus similar to both electrolytes (salts) and polymers (high molecular weight compounds). Also many biological molecules (such as, e.g., polypeptides and nucleic acids) are polyelectrolytes. It is understood that the "polyelectrolyte particle" has a positive net charge in aqueous solution and thus represents a "cationic particle", which is able to form a complex with an anionic substance (such as nucleic acids). It is understood that at least some of the repeating units of the the polymer arms comprised by the polyelectrolyte particle according to the invention comprise positively charged nitrogen atoms (also designated as "quaternized nitrogen atoms").

In the context of the present invention, the term "polyelectrolyte particle" (also designated as "cationic particle" or "cationic polymer") thus refers to a water soluble particle which in an aqueous solution, generally at physiological pH, has a positive net charge. In order to be able to bind an anionic substance, the polyelectrolyte particle according to the present invention should possess a positive charge at physiological pH. For instance, and without being limiting, the polyelectrolyte particle should be able to bind and condense nucleic acids such as DNA, e.g. in the form of a plasmid, wherein a gene construct is incorporated.

An important microstructural feature determining polymer properties is the polymer architecture. The simplest polymer architecture is a linear chain: a single backbone with no branches. A related unbranching architecture is a "ring polymer". A "branched polymer" molecule is composed of a main chain with one or more substituent side chains or branches. Special types of branched polymers include star polymers, comb polymers, brush polymers, dendronized polymers, ladders, and dendrimers.

The architecture of the polymer chains is often physically determined by the functionality of the monomers from which it is formed. This property of a monomer is defined as the number of reaction sites at which may form chemical covalent bonds. The basic functionality required for forming even a linear chain is two bonding sites. Higher functionality yields branched or even crosslinked or networked polymer chains. In an optional embodiment, the monomer units comprised by the polymer chains of the polyelectrolyte particle each contain two bonding sites.

In particular, when used herein, the term "graft polymer" denotes a branched polymer molecule in which one or more the side chains are different, structurally or configurationally, from the main chain. The term "star polymer" (or star-shaped polymer") denotes a branched polymer molecule in which a single branch point gives rise to multiple linear chains or arms. If the arms are identical the star polymer molecule is said to be "regular". If adjacent arms are composed of different repeating subunits, the star polymer molecule is said to be "variegated". In the context of the present invention, a "comb polymer" molecule consists of a main chain with two or more three-way branch points and linear side chains. When used herein, the term "brush polymer" denotes a molecule consisting of a main chain with linear, unbranched side chains and where one or more of the branch points has four-way functionality or larger. A "polymer network", as used herein, denotes a network in which all polymer chains are interconnected to form a single macroscopic entity by many crosslinks.

Branching of polymer chains affects the ability of chains to slide past one another by altering intermolecular forces, in turn affecting bulk physical polymer properties. The effect of such long-chain branches on the size of the polymer in solution is characterized by the "branching index". Dendrimers are a special case of polymer where every monomer unit is branched. Alternatively, dendritic polymers are not perfectly branched but share similar properties to dendrimers due to their high degree of branching.

In an optional embodiment of the invention, the polymer chains are linear polymer chains and the polyelectrolyte particle does not comprise any branched or cross-linked polymer chains.

A means of expressing the length of a polymer chain is the degree of polymerization, which quantifies the number of monomer units incorporated into the chain. As with other molecules, a polymer's size may also be expressed in terms of molecular weight. It is understood that since synthetic polymerization techniques typically yield a polymer product including a range of molecular weights, the weight is expressed statistically to describe the distribution of chain lengths present in the same. Common examples are the number average molecular weight and weight average molecular weight. The ratio of these two values is the polydispersity index, used to express the "width" of the molecular weight distribution. A final measurement is contour length, which can be understood as the length of the chain backbone in its fully extended state. It is understood that the polymer chains comprised by the polyelectrolyte particle according to the invention comprises at least 50 covalently linked monomer units on average. The size distribution can be characterized statistically by its weight average molecular weight ($M_w$) and its number average molecular weight ($M_n$), the ratio of

which is called the polydispersity index ($M_w/M_n$). The measure of $M_w$ and $M_n$ and the determination of the polydispersity index is routine to the person skilled in the art.

In particular, when used herein, the term "polymer chain" can denote a homopolymer or a copolymer. In the context of the present invention, monomer units within a copolymer may be organized along the backbone in a variety of ways:

(1) -A-A-A-A-A-A-A-A-A-A-
(2) -A-B-A-B-A-B-A-B-A-B-
(3) -A-B-B-B-A-B-A-B-A-A-
(4) -B-B-B-B-B-A-A-A-A-A-

(1) depicts a typcial homopolymer. "Alternating copolymers" possess regularly alternating monomer residues: $[AB...]_n$ (2). "Periodic copolymers" have monomer residue types arranged in a repeating sequence: $[A_nB_m...]$, m being different from n. "Statistical copolymers" have monomer residues arranged according to a known statistical rule. A statistical copolymer in which the probability of finding a particular type of monomer residue at an particular point in the chain is independent of the types of surrounding monomer residue is referred to as a truly random copolymer (3). "Block copolymers" have two or more homopolymer subunits linked by covalent bonds (4). Polymers with two or three blocks of two distinct chemical species (e.g., A and B) are called "diblock copolymers" and "triblock copolymers", respectively. Polymers with three blocks, each of a different chemical species (e.g., A, B, and C) are termed "triblock terpolymers". In an optional embodiment, the polymer chains comprised by the polyelectrolyte particle are homopolymers (1) or diblock copolymers (4).

Furthermore, in the context of the present invention, the polymer chains comprised by the polyelectrolyte particle may be regular (i.e. adjacent arms are composed of substantially the same repeating subunits or polymer blocks) or may be variegated (i.e. adjacent arms are composed of different repeating subunits or polymer blocks).

It is understood that there are multiple conventions for naming polymer substances. Many commonly used polymers, such as those found in consumer products, are referred to by a common or trivial name. The trivial name is assigned based on historical precedent or popular usage rather than a standardized naming convention. Both the American Chemical Society (ACS) and IUPAC have proposed standardized naming conventions; the ACS and IUPAC conventions are similar but not identical. As used herein, the polymers' names are intended to reflect the monomer unit (s) from which they are synthesized rather than the precise nature of the repeating subunit.

Examples of polymer chains comprised by the polyelectrolyte particle according to the invention are polymers (i.e. homopolymers or copolymers) comprising polyethylenglycols, polyoxazolines, optionally substituted poly(alk)acrylates, optionally substituted poly(alk)acrylamides, polylysines, and polyethyleneimines. It is however understood that at least some of the repeating units of the polymer arms comprised by the polyelectrolyte particle according to the invention contain positively charged nitrogen atoms.

In the context of the present invention, at least some of the repeating units of the polymer arms comprised by the polyelectrolyte particle according to the invention can be polyethylenglycol. Polyethylene glycol (PEG) is a polyether compound with many applications from industrial manufacturing to medicine. It has also been known as polyethylene oxide (PEO) or polyoxyethylene (POE), depending on its molecular weight. As used herein, PEG, PEO, or POE refers to a polymer of ethylene oxide. The three names are chemically synonymous, but historically PEG has tended to refer to oligomers and polymers with a molecular mass below 20,000 g/mol, PEO to polymers with a molecular mass above 20,000 g/mol, and POE to a polymer of any molecular mass. Different forms of PEG are also available dependent on the initiator used for the polymerization process, the most common of which is a monofunctional methyl ether PEG (methoxypoly(ethylene glycol)), abbreviated mPEG. PEG or PEO as used in the polymer arms according to the invention has the following chemical structure: $-(CH_2-CH_2-O)_n-$, wherein n indicates the number of repeating units. According to an optional embodiment of the invention, the polymer arms are diblock copolymers having a homopolymer subunit of PEG or PEO linked by covalent bonds to a further homopolymer subunit.

In the context of the present invention, at least some of the repeating units of the polymer arms comprised by the polyelectrolyte particle according to the invention can be polyoxazoline. In the context of the present invention, polyoxazoline polymers are referred to as products of the cationic ring-opening polymerization of optionally substituted oxazolines.. The polymers so prepared have at least two oxazoline or oxazine functionalities and can be employed in applications where a compound having an oxazoline or oxazine functionality has useful activity. According to an optional embodiment of the invention, the polymer arms are diblock copolymers having a homopolymer subunit of polyoxazoline linked by covalent bonds to a further homopolymer subunit.

In the context of the present invention, a typical example of polymerization can be the ring-opening polymerization of 2-oxazolines. 2-Oxazolines are five-membered cyclic imino ethers (imidates), which are heterocyclic compounds with an imino ether linkage. Cationic ring-opening polymerization of such cyclic imino ethers generally involves a thermodynamically favorable isomerization of the imino ether group to the amide. Suitable monomers for such polymerization

reactions include:

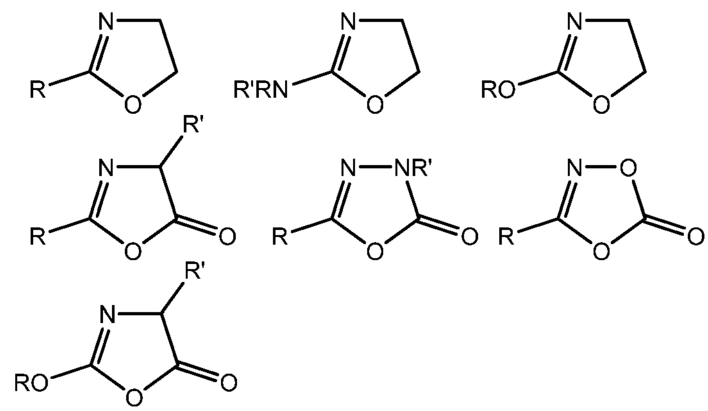

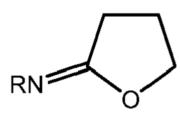
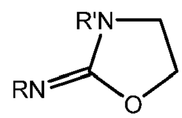
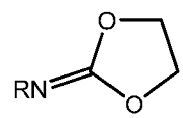
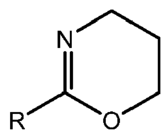
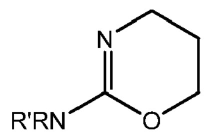
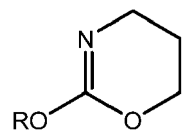
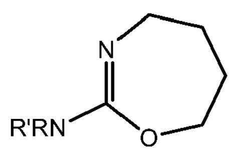

,

wherein each R and R' are independently selected from -H, -phenyl, -($C_1$-$C_6$)alkyl or -($C_3$-$C_7$)cycloalkyl, each of which is unsubstituted or substituted with 1 or 2 independently selected $R_2$ groups;
each $R_2$ is independently selected from -C(=O)N($T_3$), -N($T_1$)($T_2$), -N($T_3$)C(=O)$T_3$, -N($T_3$)C(=O)O$T_3$, or -N($T_3$)C(=O)N($T_1$)($T_2$); and
each $T_1$, $T_2$, and $T_3$ is independently selected from -H, $-CH_3$ or $-CH_2CH_3$-
The synthesis of such monomers is known to the person skilled in the art (see, e.g., K. Aoi and M. Okada (1996), Prog. Polym. Sci 21: 151-208).

Various kinds of cationic initiators have been used for the polymerization of 2-oxazolines. In the context of the present invention, types of initiators for the polymerization of cyclic imino ethers include sulfonate esters (such as p-$MeC_6H_4SO_3Me$, p-$O_2NC_6H_4SO_3Me$, $CF_3SO_3Me$, $FSO_3Me$), sulfate esters (such as $(MeO)_2SO_2$), sulfonic anhydrides (such as $MeSO_2)_2O$), alkyl halides (such as $PhCH_2Cl$), protonic acids (such as $HClO_4$, CF3SO3H, $H_2SO_4$, HBr), Lewis acids ($BF_3$, $AlCl_3$, $TiCl_4$, $PF_5$, $SbF_5$), salts of Lewis acids (such as $Et_3O^+BF_4^-$), alkyl haloformates, oxazolinium salts and electron acceptors. The selection of suitable initiators as well as the selection of suitable polymerization methods is within the knowledge of the skilled person (see, e.g., K. Aoi and M. Okada (1996), Prog. Polym. Sci 21: 151-208). According to an optional embodiment of the invention, the polymer chains diblock copolymers based on 2-methyl-2-oxazoline (MeOx) and 2-phenyl-2-oxazoline (PhOx). It is well known in the art that poly(2-oxazoline)s can be used as precursor for the synthesis of linear poly(ethylene imine) (PEI) via acidic or basic hydrolysis (see, e.g., H. M. L. Lambermont-Thijs et al. (2011), Polym. Chem. 2: 313-322).

In the context of the present invention, at least some of the repeating units of the polymer arms comprised by the polyelectrolyte particle according to the invention can be polycaprolactone. In the context of the present invention, polycaprolactone polymers are referred to as aliphatic polyesters composed of hexanoate repeat units. According to an optional embodiment of the invention, the polymer arms are diblock copolymers having a homopolymer subunit of polycaprolactone linked by covalent bonds to a further homopolymer subunit. The synthesis of such polycaprolactones is known to the person skilled in the art (see, e.g., M. Labet and W. Thielemans (2009), Chem. Soc. Rev. 38:3484-3504; being incorporated herein by reference).

In the context of the present invention, at least some of the repeating units of the polymer arms comprised by

the polyelectrolyte particle according to the invention can be polylysine. Polylysine (ε-poly-L-lysine, EPL) is a small natural homopolymer of the essential amino acid L-lysine that is produced by bacterial fermentation. Polylysine as used in the polymer arms according to the invention has the following chemical structure: $-(C(=O)-CH(NH_2)-(CH_2)_4NH)_n-$, wherein n indicates the number of repeating units. According to an optional embodiment of the invention, the polymer arms are diblock copolymers having a homopolymer subunit of polylysine linked by covalent bonds to a further homopolymer subunit.

In the context of the present invention, at least some of the repeating units of the polymer arms comprised by the polyelectrolyte particle according to the invention can be polyethyleneimine (PEI). Linear polyethyleneimines (PEIs) contain only secondary amines, in contrast to branched PEIs which contain primary, secondary and tertiary amino groups. Linear PEI as used in the polymer arms according to the invention has the following chemical structure: $-(CH_2-CH_2-NH)_n-$, wherein n indicates the number of repeating units. According to an optional embodiment of the invention, the polymer arms are diblock copolymers having a homopolymer subunit of linear PEI linked by covalent bonds to a further homopolymer subunit. According to a further optional embodiment of the invention, the polymer arms are diblock copolymers having a homopolymer subunit of branched PEI linked by covalent bonds to a further homopolymer subunit.

In a specic embodiment of the present invention, the polymer chains are each formed at least in part by the polymerisation of monomers are selected from the group consisting of APMA (3-aminopropyl methacrylamide), Boc-AEMA (N-tert-butoxycarbonyl-aminoethyl methacrylate), Boc-AEAEMA (N,N'-di-(tert-butoxycarbonyl)-2-(2-aminoethylamino)ethyl methacrylate), DEAEMA (2-(diethylamono)ethyl methacrylate), DMAEMA (2-methylaminoethyl methacrylate), DMAPMAA (N-(3-(dimethylamino)propyl) methacrylamide), DPAEMA (2-(diisopropylamino)ethyl methacrylate), MeDMA (2-(methacryloyloxy)ethyl) trimethyl ammonium chloride), HAEAPMA (2-hydroxy-3-(2-aminoethyl)amino)propyl methacrylate), BMA (butyl methacrylate), GAPMA (3-gluconamidopropyl methacrylamide), GMA (glycidyl methycrylate), HEMA (2-hydroxyethyl methacrylate), HPMA (N-(2-hydroxypropyl)methacrylamide), LAEMA (2-lactobionamidoethyl methacrylamide), MAS (methyacryloxysuccinimide), MPC (2-methacryloyloxyethyl phosphorylcholine), NIPAAm (N-isopropylacrylamide), and OEGMA (oligo(ethylene glycol) methacrylate),). The synthesis of these polymer chains can be conducted by using controlled polymerization methods such as either reversible addition-fragmentation chain transfer or atom transfer radical polymerization. The selection of a suitable sythesis method and the provision of such polymer chains is known to the skilled person.

In the context of the present invention, at least some of the repeating units of the polymer arms comprised by the polyelectrolyte particle according to the invention generally, but not necessarily, are optionally substituted poly(alk) acrylates or optionally substituted poly(alk)acrylamides. More in particular, a specific embodiment of the invention relates to water soluble or water dispersible polyacrylate, polyacrylamide, polymethacrylate, polymethacrylamide, poly(alkyl) methacrylate, poly(alkyl)methacrylamide, poly(alkyl)acrylate or poly(alkyl)acrylamide based transfection systems, wherein cationic moieties are attached to the polyacrylate, poly(alkyl)methacrylate, polymethacrylate or poly(alkyl)acrylate backbone or the backbone of the corresponding acrylamides.

In the context of the present invention, the term "(alk)acrylate" refers to the ester of 2-alkyl-2-propenoic acid, and more specifically to the ester of 2-methyl-2-propenoic acid, 2-ethyl-2-propenoic acid, or 2-propyl-2-propenoic acid. In the context of the present invention, the term "(alk)acrylamide" refers to the amide of 2-alkyl-2-propenoic acid, and more specifically to the ester of 2-methyl-2-propenoic acid, 2-ethyl-2-propenoic acid, or 2-propyl-2-propenoic acid. The terms "poly(alk)acrylate" or "poly(alk)acrylamide" denote polymers which are each formed at least in part by the polymerisation of ethylenically unsaturated esters or amides of 2-alkyl-2-propenoic acid, respectively.

According to a specific embodiment of the present invention, the polymer chains are each formed at least in part by the polymerisation of ethylenically unsaturated monomers selected from the group consisting of dialkylamino alkyl(alk)acrylates, dialkylamino alkyl(alk)acrylamides, hydroxy alkyl(alk)acrylates, hydroxy alkyl(alk)acrylamides, alkyl (alk)acrylates and alkyl(alk)acrylamides.

According to an optional embodiment, the polymer chains are each formed at least in part by the polymerisation of ethylenically unsaturated monomers comprising monomers having the following general formula (I):

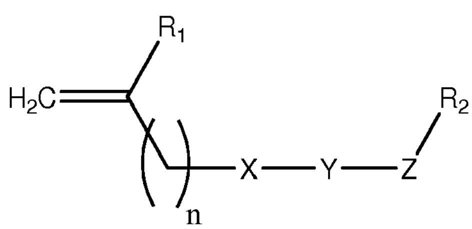

(I)

wherein:

$R_1$ is selected from hydrogen or optionally substituted -($C_1$-$C_3$)alkyl;
X is selected from -O-C(O)-, -C(O)-O-, -$NT_3$-C(O)- or -C(O)-$NT_3$-;
Y is -$(CH_2)_m$-;
Z is selected from -O-, -(O-$CH_2CH_2$)$_o$-, -($CH_2CH_2$-O)$_o$-, -O-C(O)-, -C(O)-O-, -$NT_3$-, - $NT_3$-C(O)- or -C(O)-$NT_3$-, or is absent;
n is an integer selected from 0, 1, or 2;
m is an integer selected from 0, 1, 2, 3, 4, or 5;
o is an integer selected from 1 to 10;
$R_2$ is selected from -H or -($C_1$-$C_3$)alkyl which is unsubstituted or substituted with 1 or 2 independently selected $R_4$ groups;
each $R_4$ is independently selected from -$OT_3$, -N($T_1$)($T_2$), -N($T_3$)C(=O)$T_3$, or -N($T_3$)C(=O)O$T_3$; and
each $T_1$, $T_2$, and $T_3$ is independently selected from -H or -($C_1$-$C_3$)alkyl.

According to a further optional embodiment, the polymer chains are each formed at least in part by the polymerisation of ethylenically unsaturated monomers comprising monomers having a general formula selected from the group consisting of formulae (II), (III) and (IV), wherein formulae (II), (III) and (IV) have the following chemical structures:

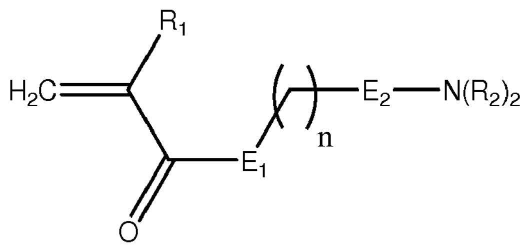
(II),

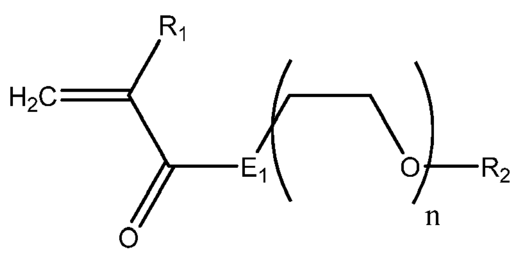
(III),

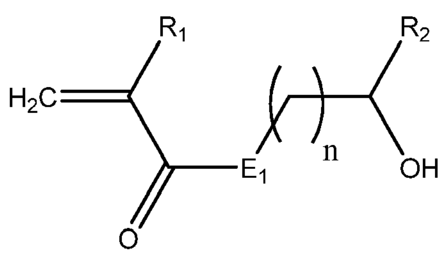
(IV),

wherein:

in formula (II):

$R_1$ is selected from -H, -$CH_3$ or -$CH_2CH_3$;
$E_1$ is selected from -O-, -NH-, or -N($CH_3$)-;
n is an integer selected from 0, 1 or 2;
$E_2$ is selected from -(NH-$CH_2$)$_m$-, -($CH_2$-NH)$_m$-, -(NH-$CH_2CH_2$)$_m$-, -($CH_2CH_2$-NH)$_m$-, -(N($CH_3$)-$CH_2$)$_m$-, -($CH_2$-N($CH_3$))$_m$-, -(N($CH_3$)-$CH_2CH_2$)$_m$-, or -($CH_2CH_2$-N($CH_3$))$_m$-, or is absent;
m is an integer selected from 0, 1, 2, 3, 4, 5, or 6; and
$R_2$ is selected from -H, -$CH_3$ and -$CH_2CH_3$;

in formula (III):

$R_1$ is selected from -H, $-CH_3$ or $-CH_2CH_3$;
$E_1$ is selected from -O-, -NH-, or $-N(CH_3)-$;
n is an integer selected from 1, 2, 3, 4, 5, 6, 7, 8, 9, or 10; and
$R_2$ is selected from -H, $-CH_3$ and $-CH_2CH_3$;

in formula (IV):

$R_1$ is selected from -H, $-CH_3$ or $-CH_2CH_3$;
$E_1$ is selected from -O-, -NH-, or $-N(CH_3)-$;
n is an integer selected from 0, 1, 2, or 3; and
$R_2$ is selected from -H, $-CH_3$ and $-CH_2CH_3$.

According to a further optional embodiment, the polymer chains are each formed at least in part by the polymerisation of ethylenically unsaturated monomers comprising monomers having a general formula selected from the group consisting of formulae (V), (VI), (VII) and (VIII), wherein formulae (V), (VI), (VII) and (VIII) have the following chemical structures:

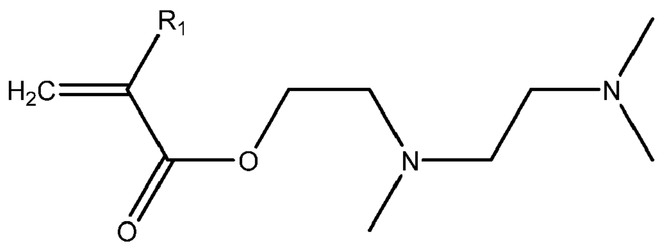

(V),

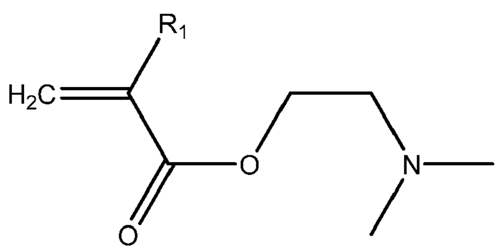

(VI),

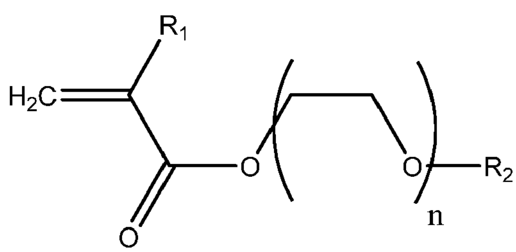

(VII),

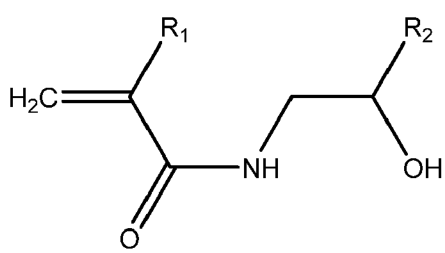

(VIII),

wherein:

in formula (V):

$R_1$ is selected from -H, $-CH_3$ or $-CH_2CH_3$;

in formula (VI):

$R_1$ is selected from -H, $-CH_3$ or $-CH_2CH_3$;

in formula (VII):

$R_1$ is selected from -H, $-CH_3$ or $-CH_2CH_3$;
n is an integer selected from 1, 2, 3, 4, 5, 6, 7, 8, 9, or 10; and
$R_2$ is selected from -H, $-CH_3$ and $-CH_2CH_3$;

in formula (VIII):

$R_1$ is selected from -H, $-CH_3$ or $-CH_2CH_3$; and
$R_2$ is selected from -H, $-CH_3$ and $-CH_2CH_3$-

According to a further optional embodiment, the polymer chains are each formed at least in part by the polymerisation of ethylenically unsaturated monomers having the general formula (VI), wherin $R_1$ is defined as above.

According to a very specific embodiment, the polymer chains are each formed by the polymerisation of ethylenically unsaturated monomers having the general formula (VI), wherin $R_1$ is defined as above. According to a further very specific embodiment, the polymer chains are each formed by the polymerisation of DMAEMA ((2-dimethylamino) ethyl methacrylate).

As used in connection with the polymers herein, the terms used herein having following meaning:

When a first group is "substituted with one or more" second groups, one or more hydrogen atoms of the first group is replaced with a corresponding number of second groups. When the number of second groups is two or greater, each second group can be the same or different. In one embodiment, a first group is substituted with up to three second groups. In another embodiment, a first group is substituted with one or two second groups. In another embodiment, a first group is substituted with only one second group.

"-($C_1$-$C_6$)alkyl" means a straight chain or branched non-cyclic hydrocarbon having from 1 to 6 carbon atoms. Representative straight chain -($C_1$-$C_6$)alkyls include -methyl, -ethyl, - n-propyl, -n-butyl, -n-pentyl, and -n-hexyl. Representative branched -($C_1$-$C_6$)alkyls include -*iso*-propyl, -*sec*-butyl, -*iso*-butyl, -*tert*-butyl, -*iso*-pentyl, -neopentyl, 1-methylbutyl, 2-methylbutyl, 3-methylbutyl, 1,1-dimethylpropyl, 1,2-dimethylpropyl, 1-methylpentyl, 2-methylpentyl, 3-methylpentyl, 4-methylpentyl, 1-ethylbutyl, 2-ethylbutyl, 3-ethylbutyl, 1,1-dimethtylbutyl, 1,2-dimethylbutyl, 1,3-dimethylbutyl, 2,2-dimethylbutyl, 2,3-dimethylbutyl, and 3,3-dimethylbutyl.

"-($C_3$-$C_7$)cycloalkyl" means a saturated monocyclic hydrocarbon having from 3 to 7 carbon atoms. Representative ($C_3$-$C_7$)cycloalkyls include cyclopropyl, -cyclobutyl, -cyclopentyl, -cyclohexyl, -and cycloheptyl.

"-($C_1$-$C_4$)alkyl" means a straight chain or branched non-cyclic hydrocarbon having from 1 to 4 carbon atoms. Representative straight chain -($C_1$-$C_4$)alkyls include -methyl, -ethyl, - n-propyl, and -n-butyl. Representative branched -($C_1$-$C_4$)alkyls include -*iso*-propyl, -*sec*-butyl, -*iso*-butyl, and -*tert*-butyl.

"-($C_1$-$C_3$)alkyl" means a straight chain or branched non-cyclic hydrocarbon having from 1 to 3 carbon atoms. Representative straight chain -($C_1$-$C_3$)alkyls include -methyl, -ethyl, and - n-propyl. Representative branched -($C_1$-$C_3$) alkyls include -*iso*-propyl.

"-($C_1$-$C_2$)alkyl" means a straight chain non-cyclic hydrocarbon having 1 or 2 carbon atoms. Representative straight chain -($C_1$-$C_2$)alkyls include -methyl and -ethyl.

"-(5- or 6-membered)heteroaryl" means a monocyclic aromatic heterocycle ring of 5 or 6 members where at least one carbon atom is replaced with a heteroatom independently selected from nitrogen, oxygen, and sulfur. In one

embodiment, one of the -(5- or 6-membered)heteroaryl's ring contains at least one carbon atom. Representative -(5- or 6-membered)heteroaryls include pyridyl, furyl, pyrrolyl, oxazolyl, imidazolyl, thiazolyl, isoxazolyl, 1,2,3-oxadiazolyl, 1,3,4-oxadiazolyl, 1,2,5-oxadiazolyl, 1,2,3-triazolyl, pyrazolyl, isothiazolyl, pyridazinyl, pyrimidyl, pyrazinyl, 1,2,3-thiadiazolyl, 1,3,4-thiadiazolyl, 1,2,5-thiadiazolyl, 1,3,5-triazinyl, and thiophenyl.

"$-CH_2$(halo)" means a methyl group where one of the hydrogens of the methyl group has been replaced with a halogen. Representative $-CH_2$(halo) groups include $-CH_2F$, $-CH_2Cl$, $-CH_2Br$, and $-CH_2I$.

"-CH(halo)$_2$" means a methyl group where two of the hydrogens of the methyl group have been replaced with a halogen. Representative -CH(halo)$_2$ groups include $-CHF_2$, $-CHCl_2$, - $CHBr_2$, -CHBrCl, -CHClI, and $-CHI_2$.

"-C(halo)$_3$" means a methyl group where each of the hydrogens of the methyl group has been replaced with a halogen. Representative -C(halo)$_3$ groups include $-CF_3$, $-CCl_3$, $-CBr_3$, and - $CI_3$.

"-Halogen" or "-halo" means -F, -Cl, -Br, or -I.

"Oxo", "=O", and the like as used herein mean an oxygen atom doubly bonded to carbon or another element.

"Thiooxo", "thioxo", "=S", and the like as used herein mean a sulfur atom doubly bonded to carbon or another element.

"Imino", "$=NT_3$", and the like as used herein mean a nitrogen atom doubly bonded to carbon or another element.

As used herein in connection with the polyelectrolyte particle, if the polymer chains are each formed at least in part by the polymerisation of ethylenically unsaturated monomers having the general formula (I), then this part of the polymer chains is understood to appear as follows:

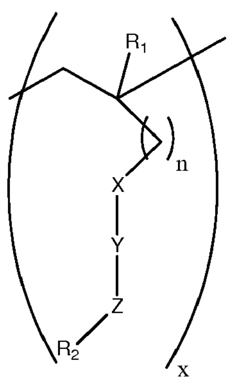

,

wherein $R_1$, n, X, Y, Z and $R_2$ are defined as herein above, and x denotes the (average) number of covalently linked monomer units comprised by each polymer chain.

As used herein in connection with the polyelectrolyte particle, if the polymer chains are each formed at least in part by the polymerisation of ethylenically unsaturated monomers having the general formula (II), then this part of the polymer chains is understood to appear as follows:

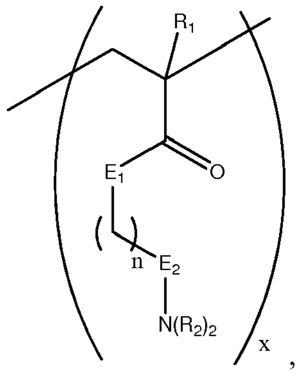

wherein $R_1$, n, $E_1$, $E_2$ and $R_2$ are defined as herein above, and x denotes the (average) number of covalently linked monomer units comprised by each polymer chain.

As used herein in connection with the polyelectrolyte particle, if the polymer chains are each formed at least in part by the polymerisation of ethylenically unsaturated monomers having the general formula (III), then this part of the polymer chains is understood to appear as follows:

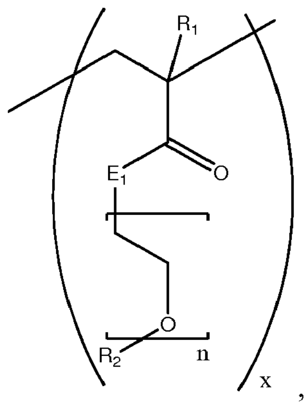

wherein $R_1$, n, $E_1$, and $R_2$ are defined as herein above, and x denotes the (average) number of covalently linked monomer units comprised by each polymer chain.

As used herein in connection with the polyelectrolyte particle, if the polymer chains are each formed at least in part by the polymerisation of ethylenically unsaturated monomers having the general formula (IV), then this part of the polymer chains is understood to appear as follows:

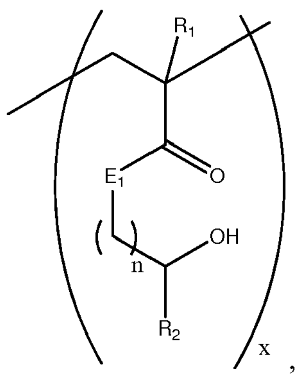

wherein $R_1$, n, $E_1$, and $R_2$ are defined as herein above, and x denotes the (average) number of covalently linked monomer units comprised by each polymer chain.

As used herein in connection with the polyelectrolyte particle, if the polymer chains are each formed at least

in part by the polymerisation of ethylenically unsaturated monomers having the general formula (V), then this part of the polymer chains is understood to appear as follows:

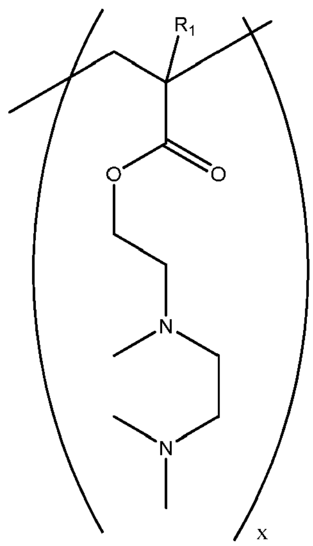

,

wherein $R_1$ is defined as herein above, and x denotes the (average) number of covalently linked monomer units comprised by each polymer chain.

As used herein in connection with the polyelectrolyte particle, if the polymer chains are each formed at least in part by the polymerisation of ethylenically unsaturated monomers having the general formula (VI), then this part of the polymer chains is understood to appear as follows:

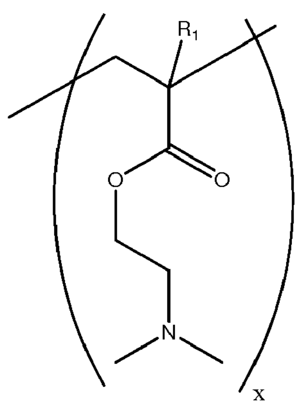

,

wherein $R_1$ is defined as herein above, and x denotes the (average) number of covalently linked monomer units comprised by each polymer chain.

As used herein in connection with the polyelectrolyte particle, if the polymer chains are each formed at least in part by the polymerisation of ethylenically unsaturated monomers having the general formula (VII), then this part of the polymer chains is understood to appear as follows:

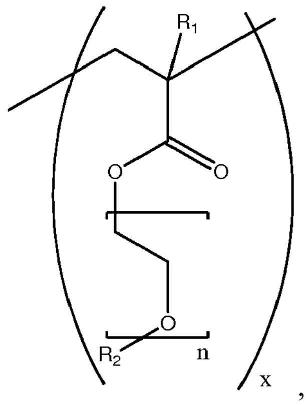

wherein $R_1$, n and $R_2$ are defined as herein above, and x denotes the (average) number of covalently linked monomer units comprised by each polymer chain.

As used herein in connection with the polyelectrolyte particle, if the polymer chains are each formed at least in part by the polymerisation of ethylenically unsaturated monomers having the general formula (VIII), then this part of the polymer chains is understood to appear as follows:

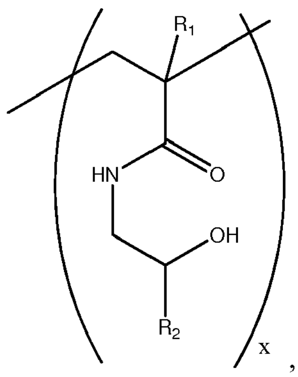

wherein $R_1$, n, $E_1$, and $R_2$ are defined as herein above, and x denotes the (average) number of covalently linked monomer units comprised by each polymer chain.

As used herein in connection with the polyelectrolyte particle, if the polymer chains are each formed at least in part by the polymerisation of DMAEMA, then this part of the polymer chains is understood to appear as follows:

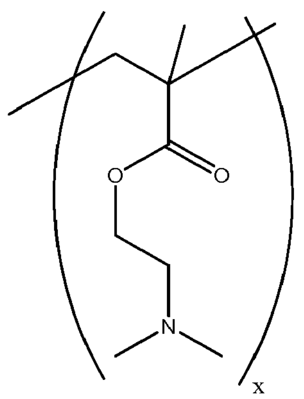

wherein $R_1$ is defined as herein above, and x denotes the (average) number of covalently linked monomer units comprised by each polymer chain.

According to an optional embodiment, the polymer chains can also be diblock copolymers having a homopolymer subunit of linear PEI linked by covalent bonds to a further homopolymer subunit formed by the polymerisation of ethylenically unsaturated monomers selected from the group consisting of monomers having the general formulae (I), (II), (III), (IV), (V), (VI), (VII), and (VIII).

According to an optional embodiment, the polymer chains can also be diblock copolymers having a homopolymer subunit formed by the polymerisation of ethylenically unsaturated monomers selected from the group consisting of monomers having the general formulae (I), (II), (III), (IV), (V), (VI), (VII), and (VIII), which subunit is linked by covalent bonds to a different homopolymer subunit formed by the polymerisation of ethylenically unsaturated monomers selected from the group consisting of monomers having the general formulae (I), (II), (III), (IV), (V), (VI), (VII), and (VIII).

According to an optional embodiment, the polymer chains can also be diblock copolymers having a homopolymer subunit of PEG linked by covalent bonds to a further homopolymer subunit.

According to an optional embodiment, the polymer chains can also be diblock copolymers having a homopolymer subunit formed by the polymerisation of butadiene, which subunit is linked by covalent bonds to a further homopolymer subunit formed by the polymerisation of ethylenically unsaturated monomers selected from the group consisting of monomers having the general formulae (I), (II), (III), (IV), (V), (VI), (VII), and (VIII).

According to a specific embodiment, the polymer chains can be a block copolymer, werein at least one block is selected from a biodegradable polyester (such as, e.g. polylactide, polyglycolide, polycaprolactone etc.). According to a further specific embodiment, the polymer chains can be a block copolymer, werein at least one block is a hydrophobic polymer formed by the polymerisation of a monomer selected from the group consisting of butadiene, isoprene, methylmethacrylate, N-butylacrylate, polystyrole, and polycaprolactone. Such hydrophobic polymers are known to the skilled person (see, e.g., G. Riess (2003), Prog. Polym. Sci. 28:1107-1170; being incorporated herein by reference).

According to a specific embodiment, the polymer chains can also be a diblock copolymer selected from the group consisting of PEG-*block*-PAEMA, PEG-*block*-PDEAEMA, PEG-*block*-PDMAEMA, PEG-*block*-P(DMAPMAA-co-(HEMA-PCL)), PEG-*block*-Aminated PGMA, PMPC-*block*-PDEAEMA, PMPC-*block*-PDMAEMA, PMPC-*block*-PDPAEMA, PEEP-*block*-PDMAEMA, PHPMA-*block*-PDMAPMAA, P(HPMA-co-APMA)-*block*-PDMAPMAA, PAPMA-*block*-PGAPMA, PAPMA-*block*-PLAEMA, PDMAEMA-*block*-PBMA, PDMAEMA-*block*-P(DMAEMA-co-PAA-co-BMA), and aminated PtBA-*block*-PS. The synthesis of these block copolymers is known to the skilled person (see, e.g., FJ Xu, WT Yang (2011), Prog. Polym. Sci, doi: 10.1016/j.progpolymsci.2010.11.005).

According to a specific embodiment, the polymer chains can also be a triblock copolymer selected from the group consisting of PDMAEMA-*block*-PEG-*block*-PDMAEMA, PEG-*block*-PDMAEMA-*block*-PEG, PEG-*block*-PDPAEMA-block-PDMAEMA, PEG-*block*-PBMA-*block*-PDMAEMA, PEG-*block*-PCL-*block*-PDMAEMA, and PDMAEMA-*block*-PCL-*block*-PDMAEMA. The synthesis of these block copolymers is known to the skilled person (see, e.g., FJ Xu, WT Yang (2011), Prog. Polym. Sci, doi: 10.1016/j.progpolymsci.2010.11.005). Further pentablock copolymers are known to the skilled person (see, e.g., FJ Xu, WT Yang (2011), Prog. Polym. Sci, doi: 10.1016/j.progpolymsci.2010.11.005).

Generally speaking, star polymers can be synthesized via one of three common strategies: "core-first" by growing arms from a multifunctional initiator; "coupling-onto" by attaching linear polymer arm precursors onto a multifunctional core and the "arm-first" method by cross-linking preformed linear polymer arm precursors using a divinyl compound. Suitable synthesis strategies are well known to the person skilled in the art (see, e.g., K. Matyjaszewski, Y. Gnanou, L. Leibler, Eds.: "Macromolecular Engineering" (4 volumes), Wiley-VCH, Weinheim 2007; LV Vinogradova (2010), Russian Journal of Applied Chemistry 83(3), 351-378; H Gao et al. (2009), Progress in Polymer Science 34, 317-350; which publications are incorporated herein by reference).

In particular, the principles of controlled radical polymerization (CRP) are well-known to a person skilled in the art (e.g., see H Gao et al. (2009), Progress in Polymer Science 34, 317-350; being incorporated herein by reference) and can be employed for the synthesis of polyelectrolyte particles according to the invention. In CRP, the fast initiation reactions, relative to propagation reactions, result in all polymer chains undergoing initiation at approximately the same time and a nearly constant number of chains growing throughout the polymerization, which enables control over chain architecture. Three different mechanisms of intermittent activation are employed in CRPs. They include: dissociation-combination, represented by nitroxide mediated polymerization (NMP) or organometallic radical polymerization; catalytic atom (group) transfer, represented by atom transfer radical polymerization (ATRP); and degenerative chain transfer, represented by iodine mediated polymerization or reversible addition-fragmentation chain transfer (RAFT) polymerization. During these CRP processes, the active radicals either undergo a reversible activation/deactivation process (i.e., NMP and ATRP), or participate in a degenerative transfer reaction (e.g., RAFT) to assure simultaneous growth of all polymer chains. The ability of CRP techniques to control molecular weight and polydispersity and to provide access to welldefined molecular architecture, originates from both fast initiation of all chains and limitation of the chain growth during each activation cycle to a level where the contribution of chain breaking reactions is negligible.

In the context of the present invention, the term "magnetic nanoparticle" denotes matrix-dispersed nanoparticles having superparamagnetic properties. Examples of such nanoparticles are known to the skilled person (see, e.g., S. Behrens (2011), Nanoscale 3: 877; being incorporated herein by reference).

Optionally, the functionalized magnetic nanoparticle is a functionalized maghemite nanoparticle or a dopamine-functionalized maghemite nanoparticle based core. For instance, syntheses of magnetic nanoparticles of various elemental compositions and phases have been reported in the literature, e.g., $Fe_3O_4$ and $\gamma$-$Fe_2O_3$, metallic Co, Fe, Ni, spinel-type ferrite $MgFe_2O_4$, $MnFe_2O_4$, $CoFe_2O_4$ and alloyed FePt, FeCo, $CoPt_3$, or FeCoPt nanoparticles (see, e.g., S. Behrens (2011), Nanoscale 3: 877; being incorporated herein by reference). The principles of synthesis of polyelectrolyte particles having a functionalized maghemite nanoparticle (such as the FeO nanoparticle based macroinitiator of Example 2) as branching centre are well-known to a person skilled in the art (e.g., see K. Matyjaszewski, Y. Gnanou, L. Leibler, Eds.: "Macromolecular Engineering" (4 volumes), Wiley-VCH, Weinheim 2007; being incorporated herein by reference).

In particular, polymer-coated nanoparticles can be produced directly by *in situ* precipitating or thermolysing a metal precursor in the presence of the polymer which then acts as a stabilizer. Polymeric surfactants can be designed with tunable composition via copolymerization or post-functionalization techniques. Such polymeric surfactants must include a few general features, such as functional groups to anchor the particle surface (e.g., carboxylic acid, phosphine oxide, amine, or amide groups) located either along the polymeric backbone and/or at the end. In the art, a wide range of polymers has been used to date to synthesize and colloidally stabilize magnetic nanoparticles via polymeric coatings. Alternatively, the surface functionalization of magnetic nanoparticles with polymeric surfactants may be achieved by replacing the ligands used in the initial nanoparticle synthesis. The nanoparticles are synthesized separately and mixed with the polymer to enable ligand exchange by either physical or chemical adsorption of the polymer onto the magnetic material. Surface initiated polymerization allows the controllable polymerization of end-tethered polymer coronas directly from the nanoparticle surface. A key requirement of this approach is the introduction of appropriate, surface attached initiators, while suppressing undesirable flocculation of the colloid.

According to a specific embodiment of the invention, the polyelectrolyte particles according to the invention are star-shaped particles having a functionalized maghemite nanoparticle based core. Suitable synthesis strategies for such nanoparticles are well known to the person skilled in the art (see, e.g., T Hyeon et al. (2001), J. Am. Chem. Soc. 123, 12798-12801; being incorporated herein by reference). Suitable initiators are also well-known to a person skilled in the art (see, e.g., X. Fan et al. (2005), J. Am. Chem. Soc. 127, 15843-15847; being incorporated herein by reference). For example, the maghemite nanoparticles can be functionalized with a catechol-terminated dopamine-initiator in a suitable solvent. According to a very specific embodiment of the invention, the polyelectrolyte particles according to the invention are star-shaped particles having a dopamine-functionalized maghemite nanoparticle based core, wherein the polymer chains are each formed by the polymerisation of ethylenically unsaturated monomers having the general formula (VI), wherin $R_1$ is defined as above.

However, it is understood that, in principle, any magnetic nanoparticle having a suitable number of attachment sites (i.e. reactive sites / initiation sites) for the attachment / synthesis of the polymer arms can be used as branching centre in the context of the present invention. It is however understood that, due to sterical hindrance, only some of said attachment sites will be attached to polymer arms.

According to an optional embodiment, the polyelectrolyte particle comprises at least 15, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, or 90 polymer chains. According to an optional embodiment, the polyelectrolyte particle comprises at least 15, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, or 90 linear polymer chains. According to an optional embodiment, the polyelectrolyte particle comprises at least 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, or 90 polymer chains. According to an optional embodiment, the polyelectrolyte particle comprises at least 40, 42, 44, 46, 48, 50, 52, 54, 56, 58, 60, 62, 64, 66, 68, 70, 72, 74, 76, 78, 80, 82, 84, 86, 88, or 90 polymer chains. According to an optional embodiment, the polyelectrolyte particle comprises at least 50, 52, 54, 56, 58, 60, 62, 64, 66, 68, 70, 72, 74, 76, 78, 80, 82, 84, 86, 88, or 90 polymer chains. According to an optional embodiment, the polyelectrolyte particle comprises at least 60, 62, 64, 66, 68, 70, 72, 74, 76, 78, 80, 82, 84, 86, 88, or 90 polymer chains. According to an optional embodiment, the polyelectrolyte particle comprises at least 70, 72, 74, 76, 78, 80, 82, 84, 86, 88, or 90 polymer chains. According to an optional embodiment, the polyelectrolyte particle comprises at least 80, 82, 84, 86, 88, or 90 polymer chains.

According to an optional embodiment, the polyelectrolyte particle comprises between 10 and 500 polymer chains. According to an optional embodiment, the polyelectrolyte particle comprises between 25 and 450 polymer chains. According to an optional embodiment, the polyelectrolyte particle comprises between 50 and 400 polymer chains. According to an optional embodiment, the polyelectrolyte particle comprises between 15 and 180 polymer chains. According to an optional embodiment, the polyelectrolyte particle comprises between 15 and 25 polymer chains. According to an optional embodiment, the polyelectrolyte particle comprises between 40 and 150 polymer chains. According to an optional embodiment, the polyelectrolyte particle comprises between 40 and 230 polymer chains. According to an optional embodiment, the polyelectrolyte particle comprises between 15 and 300 polymer chains. According to an optional embodiment, the polyelectrolyte particle comprises between 40 and 300 polymer chains. According to an optional embodiment, the polyelectrolyte particle comprises between 80 and 300 polymer chains. According to an optional

embodiment, the polyelectrolyte particle comprises between 40 and 250 polymer chains. According to an optional embodiment, the polyelectrolyte particle comprises between 40 and 200 polymer chains.

According to a more specfic embodiment, each of the polymer chains comprised by the polyelectrolyte particle according to the invention comprises at least 50, 54, 58, 60, 64, 68, 70, 74, 78, 80, 84, 88, 90, 94, 98, 100, 104, 108, 110, 114, 118, 120, 124, 128, 130, 134, 138, 140, 144, 148, 150, 154, 158, 160, 164, 168, 170, 174, 178, 180, 184, 188, 190, 194, 198, or 200 covalently linked monomer units on average. According to a more specfic embodiment, each of the polymer chains comprised by the polyelectrolyte particle according to the invention comprises at least 70, 74, 78, 80, 84, 88, 90, 94, 98, 100, 104, 108, 110, 114, 118, 120, 124, 128, 130, 134, 138, 140, 144, 148, 150, 154, 158, 160, 164, 168, 170, 174, 178, 180, 184, 188, 190, 194, 198, or 200 covalently linked monomer units on average. According to a more specfic embodiment, each of the polymer chains comprised by the polyelectrolyte particle according to the invention comprises at least 90, 94, 98, 100, 104, 108, 110, 114, 118, 120, 124, 128, 130, 134, 138, 140, 144, 148, 150, 154, 158, 160, 164, 168, 170, 174, 178, 180, 184, 188, 190, 194, 198, or 200 covalently linked monomer units on average. According to a more specfic embodiment, each of the polymer chains comprised by the polyelectrolyte particle according to the invention comprises at least 110, 114, 118, 120, 124, 128, 130, 134, 138, 140, 144, 148, 150, 154, 158, 160, 164, 168, 170, 174, 178, 180, 184, 188, 190, 194, 198, or 200 covalently linked monomer units on average. According to a more specfic embodiment, each of the polymer chains comprised by the polyelectrolyte particle according to the invention comprises at least 150, 154, 158, 160, 164, 168, 170, 174, 178, 180, 184, 188, 190, 194, 198, or 200 covalently linked monomer units on average. According to a more specfic embodiment, each of the polymer chains comprised by the polyelectrolyte particle according to the invention comprises at least 170, 174, 178, 180, 184, 188, 190, 194, 198, or 200 covalently linked monomer units on average.

According to a specfic embodiment, each of the polymer chains comprised by the polyelectrolyte particle according to the invention comprises between 50 and 2000 covalently linked monomer units on average. According to a specfic embodiment, each of the polymer chains comprised by the polyelectrolyte particle according to the invention comprises between 60 and 1600 covalently linked monomer units on average. According to a specfic embodiment, each of the polymer chains comprised by the polyelectrolyte particle according to the invention comprises between 80 and 1400 covalently linked monomer units on average. According to a specfic embodiment, each of the polymer chains comprised by the polyelectrolyte particle according to the invention comprises between 100 and 1200 covalently linked monomer units on average.

According to a specific embodiment, each of the polymer chains comprised by the polyelectrolyte particle according to the invention comprises between 40 and 600 covalently linked monomer units on average. According to a specific embodiment, each of the polymer chains comprised by the polyelectrolyte particle according to the invention comprises between 40 and 1200 covalently linked monomer units on average. According to a specific embodiment, each of the polymer chains comprised by the polyelectrolyte particle according to the invention comprises between 80 and 600 covalently linked monomer units on average. According to a specific embodiment, each of the polymer chains comprised by the polyelectrolyte particle according to the invention comprises between 80 and 1200 covalently linked monomer units on average. According to a specific embodiment, each of the polymer chains comprised by the polyelectrolyte particle according to the invention comprises between 100 and 1000 covalently linked monomer units on average. According to a specific embodiment, each of the polymer chains comprised by the polyelectrolyte particle according to the invention comprises between 150 and 800 covalently linked monomer units on average. According to a specific embodiment, each of the polymer chains comprised by the polyelectrolyte particle according to the invention comprises between 200 and 800 covalently linked monomer units on average.

According to a specific embodiment, the polyelectrolyte particle comprises the following ranges of polymer chains (as indicated in following Table A in the top row) and each of the polymer chains comprised by the polyelectrolyte particle comprises the following ranges of covalently linked monomer units on average (as indicated in following Table A in the left column), wherein Table A explicitly discloses the specific combinations of both ranges (indicated by an "x", respectively:

Table A:

| | 15-180 | 15-25 | 40-150 | 40-230 | 15-300 | 40-300 | 80-250 |
|---|---|---|---|---|---|---|---|
| 40-600 | x | x | x | x | x | x | x |
| 40-1200 | x | x | x | x | x | x | x |
| 80-600 | x | x | x | x | x | x | x |
| 80-1200 | x | x | x | x | x | x | x |
| 100-1000 | x | x | x | x | x | x | x |

(continued)

| | 15-180 | 15-25 | 40-150 | 40-230 | 15-300 | 40-300 | 80-250 |
|---|---|---|---|---|---|---|---|
| 150-800 | x | x | x | x | x | x | x |
| 200-800 | x | x | x | x | x | x | x |

Optionally, the polymer chains comprised by the polyelectrolyte particle together have a molecular weight of at least 500 kDa, 550 kDa, 600 kDa, 650 kDa, 700 kDa, 750 kDa, 800 kDa, 850 kDa, 900 kDa, 950 kDa, 1000 kDa, 1500 kDa, 2000 kDa, 2500 kDa, 3000 kDa, 3500 kDa, 4000 kDa, 4500 kDa, 5000 kDa, 6000 kDa, 7000 kDa, 8000 kDa, 9000 kDa, 10,000 kDa, 15,000 kDa, 20,000 kDa, or 30,000 kDa.

In one embodiment, the method according to the invention comprises:

(a) contacting the polyelectrolyte particle and a eukaryotic cell or a microbioreactor, comprising the intracellular molecule, under conditions for interaction of said polyelectrolyte particle with the intracellular molecule to form a conjugate;
(b) recovering the conjugate from the eukaryotic cell or the microbioreactor; and
(c) isolating the intracellular molecule from the conjugate.

In a specific embodiment, the method according to the invention comprises:

(a) contacting the polyelectrolyte particle and a eukaryotic cell comprising the intracellular molecule, under conditions for interaction of said polyelectrolyte particle with the intracellular molecule to form a conjugate;
(b) recovering the conjugate from the eukaryotic cell; and
(c) isolating the intracellular molecule from the conjugate.

In a further specific embodiment, the method according to the invention comprises:

(a) contacting the polyelectrolyte particle and a microbioreactor comprising the intracellular molecule, under conditions for interaction of said polyelectrolyte particle with the intracellular molecule to form a conjugate;
(b) recovering the conjugate from the microbioreactor; and
(c) isolating the intracellular molecule from the conjugate.

In a specific embodiment of the invention, the intracellular molecule comprises the functionality of binding of the intracellular molecule to a ligand or antigen. In the context of the present invention, a "ligand" (from the Latin ligandum, binding) is a biological substance that forms a complex with a biomolecule to serve a biological purpose. In a narrower sense, it is a signal triggering molecule, binding to a site on a target protein. Generally, the binding occurs by intermolecular forces, such as ionic bonds, hydrogen bonds and van der Waals forces. The docking (association) is usually reversible (dissociation). As used herein, the term "ligand" includes substrates, inhibitors, activators, and neurotransmitters. In the context of the present invention, an "antigen" is characterized by its ability to be "bound" at the antigen-binding site of an antibody. Antigens are usually proteins or polysaccharides. This includes parts (coats, capsules, cell walls, flagella, fimbrae, and toxins) of bacteria, viruses, and other microorganisms.

In a further specific embodiment of the invention, the intracellular molecule comprises the functionality of exertion of a catalytic activity, optionally an enzymatic activity, by the intracellular molecule. As used herein, catalytic activity means the change in rate of a chemical reaction due to the participation of a substance called a catalyst. Unlike other reagents that participate in the chemical reaction, a catalyst is not consumed by the reaction itself. A catalyst may participate in multiple chemical transformations. Catalysts that speed the reaction are called positive catalysts. Substances that interact with catalysts to slow the reaction are called inhibitors (or negative catalysts). Optionally, the intracellular molecule can be an enzyme. As used herein, "enzymes" are proteins that catalyze (i.e., increase the rates of) chemical reactions. In enzymatic reactions, the molecules at the beginning of the process are called substrates, and they are converted into different molecules, called the products. Almost all processes in a biological cell need enzymes to occur at significant rates. Since enzymes are selective for their substrates and speed up only a few reactions from among many possibilities, the set of enzymes made in a cell determines which metabolic pathways occur in that cell. In a further specific embodiment of the invention, the intracellular molecule comprises the functionality of susceptibility of the intracellular molecule to a catalytic activity, optionally an enzymatic activity.

In a further specific embodiment of the invention, the intracellular molecule comprises the functionality of facilitation by the intracellular molecule of altered transport of the polyelectrolyte particle across a biological membrane. In a further specific embodiment of the invention, the intracellular molecule comprises the functionality of facilitation by

the intracellular molecule of altered transport of the polyelectrolyte particle in the intracellular compartment. Examples of such intracellular molecules include membrane transport proteins, vesicular transport proteins and carrier proteins.

A membrane transport protein (or simply transporter) is a membrane protein involved in the movement of ions, small molecules, or macromolecules (such as the polyelectrolyte particle) across a biological membrane. Transport proteins are integral membrane proteins; that is they exist within and span the membrane across which they transport substances. The proteins may assist in the movement of substances by facilitated diffusion or active transport. These mechanisms of action are known as carrier-mediated transport. Examples of membrane transport proteins include:

(1) channels/pores,
(2) electrochemical potential-driven transporters (such as mitochondrial membrane transport protein, glucose transporter, neurotransmitter transporters, glutamate/aspartate transporters, GABA transporters, glycine transporters, monoamine transporters (including dopamine transporter (DAT), norepinephrine transporter (NET), serotonin transporter (SERT) and vesicular monoamine transporters (VMAT)), adenosine transporters and vesicular acetylcholine transporter (VAChT)),
(3) primary Active Transporters (such as ATP-binding cassette transporter genes (including P-glycoprotein and CD98), V-ATPase, ion transporters (including $Na^+/K^+$-ATPase, plasma membrane $Ca^{2+}$ ATPase, proton pump, hydrogen potassium ATPase and sodium-chloride symporter))
(4) co-transporters (Symporters), i.e. molecules that transport two or more ions together in the same direction.

A vesicular transport protein is a transmembrane or membrane associated protein. It regulates or facilitates the movement by vesicles of the contents of the cell. Examples of vesicular transport proteins include: Archain, ARFs, Clathrin, Caveolin, Dynamin and related proteins, such as the EHD protein family, Rab proteins, SNAREs, sorting nexins, and Synaptotagmin.

Carrier proteins are integral membrane proteins; that is they exist within and span the membrane across which they transport substances. The proteins may assist in the movement of substances by facilitated diffusion or active transport. These mechanisms of movement are known as carrier mediated transport.

In a further specific embodiment of the invention, the intracellular molecule comprises the functionality of influence by the intracellular molecule on expression of at least one gene in a population of eukaryotic cells. Protein expression is a subcomponent of gene expression. It consists of the stages after DNA has been transcribed in mRNA which in turn has been translated into polypeptide chains, which are ultimately folded into proteins. Molecular biology research uses an enormous number of proteins and enzymes many of which are from expression systems; particularly DNA polymerase for PCR, reverse transcriptase for RNA analysis and restriction endonucleases for cloning.

In a further specific embodiment of the invention, the intracellular molecule comprises the functionality of influence by the intracellular molecule on the growth or metabolism of a population of eukaryotic cells. Prominent examples of such intracellular molecules are intracellular growth factors and transcription factors. A growth factor is a naturally occurring substance capable of stimulating cellular growth, proliferation and cellular differentiation. Usually it is a protein or a steroid hormone. Growth factors are important for regulating a variety of cellular processes.

In a specific embodiment of the invention, the intracellular molecule is selected from RNA, a lipid, a phospholipid, a carbohydrate, a peptide, an oligopeptide, a polypeptide, a monomeric protein and a multimeric protein.

In this context, examples of "proteins" include human growth hormone, growth hormone releasing hormone, growth hormone releasing peptide, interferons, colony stimulating factors, interleukins, macrophage activating factor, macrophage peptide, B cell factor, T cell factor, protein A, allergy inhibitor, cell necrosis glycoproteins, immunotoxin, lymphotoxin, tumor necrosis factor, tumor suppressors, metastasis growth factor, alpha-1 antitrypsin, albumin and fragment polypeptides thereof, apolipoprotein-E, erythropoietin, factor VII, factor VIII, factor IX, plasminogen activating factor, urokinase, streptokinase, protein C, C-reactive protein, renin inhibitor, collagenase inhibitor, superoxide dismutase, platelet-derived growth factor, epidermal growth factor, osteogenic growth factor, bone stimulating protein, calcitonin, insulin, atriopeptin, cartilage inducing factor, connective tissue activating factor, follicle stimulating hormone, luteinizing hormone, luteinizing hormone releasing hormone, nerve growth factors, parathyroid hormone, relaxin, secretin, somatomedin, insulin-like growth factor, adrenocortical hormone, glucagon, cholecystokinin, pancreatic polypeptide, gastrin releasing peptide, corticotropin releasing factor, thyroid stimulating hormone, monoclonal or polyclonal antibodies against various viruses, bacteria, toxins, etc., and virus-derived vaccine antigens.

As used herein, the term "amino acid" refers to a list of abbreviations, letters, characters or words representing amino acid residues. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes. The terms "polypeptide", "peptide", "oligopeptide", "polypeptide", "gene product", "expression product" and "protein" are used interchangeably herein to refer to a polymer or oligomer of consecutive amino acid residues. The term "nucleic acid" refers to deoxyribonucleotides or ribonucleotides and polymers or hybrids thereof in either single-or double-stranded, sense or antisense form.

Carbohydrates include compounds such as monosaccharides, oligosaccharides, polysaccharides, glycoproteins, glycolipids and the like. Carbohydrates of the present invention also include carbohydrate-nucleoside hybrid molecules, such as carbohydrate-oligonucleotide hybrid molecules. As used herein, the term "monosaccharide" includes a compound that is the basic unit of a carbohydrate, consisting of a single sugar. Monosaccharides include glucose, glyceraldehydes, ribose, dexoxyribose, mannose, galactose and the like. As used herein, the term "oligosaccharide" refers without limitation to several (e.g., two to ten) covalently linked monosaccharide units. Oligosaccharides include disaccharides (i.e., two monosaccharide units) such as sucrose, lactose, maltose, isomaltose, cellobiose and the like. Oligosaccharides are often associated with proteins (i.e., glycoproteins) and lipids (i.e., glycolipids). Oligosaccharides form two types of attachments to proteins: N-glycosidic and O-glycosidic. As used herein, the term "polysaccharide" refers without limitation to many (e.g., eleven or more) covalently linked monosaccharide units. Polysaccharides can have molecular masses ranging well into millions of daltons. Polysaccharides include cellulose, chitin, starch, glycogen, glycosaminoglycans (e.g., hyaluronic acid, chondroitin-4-sulfate, chondroitin-6-sulfate, dermatan sulfate, keratin sulfate, heparin and the like) and the like.

Examples of "lipids" include oils, fatty acids, monoglycerides, diglycerides, triglycerides, sorbitan fatty acid esters, phospholipids, sphingolipids, cholesterol, waxes, and salts and derivatives thereof. In the context of the present invention, "phospholipids" are a class of lipids and are a major component of all cell membranes as they can form lipid bilayers. Most phospholipids contain a diglyceride, a phosphate group, and a simple organic molecule such as choline; one exception to this rule is sphingomyelin, which is derived from sphingosine instead of glycerol. Examples of phospholipids include: Phosphatidic acid (phosphatidate) (PA), Phosphatidylethanolamine (cephalin) (PE), Phosphatidylcholine (lecithin) (PC), Phosphatidylserine (PS), Phosphatidylinositol (PI), Phosphatidylinositol phosphate (PIP), Phosphatidylinositol bisphosphate (PIP2), Phosphatidylinositol triphosphate (PIP3), Ceramide phosphorylcholine (Sphingomyelin) (SPH), Ceramide phosphorylethanolamine (Sphingomyelin) (Cer-PE) and Ceramide phosphorylglycerol.

It is understood that the step of "contacting the polyelectrolyte particle and a eukaryotic cell or a microbioreactor, comprising the intracellular molecule, under conditions for interaction of said polyelectrolyte particle with the intracellular molecule to form a conjugate" means to facilitate the transport or introduction of the polyelectrolyte particle into the eukaryotic cell or the microbioreactor. Methods of introducing or transferring such particles into cells are well known to the person skilled in the art. "Introduction or transfer" means that the polyelectrolyte particle is transferred into the cell and is located, at the end of the process, inside said cell, within its nucleus or within or on its membrane.

In a specific embodiment of the invention, the polyelectrolyte particle further comprises a nucleic acid. If the polyelectrolyte particle further comprises a nucleic acid, the step of "contacting the polyelectrolyte particle and a eukaryotic cell or a microbioreactor, comprising the intracellular molecule, under conditions for interaction of said polyelectrolyte particle with the intracellular molecule to form a conjugate" is called "transfection". As used herein, the term "transfection" denotes the process of deliberately introducing nucleic acids into eukaryotic cells. However, it may also refer to other methods and cell types. In particular, genetic material (such as supercoiled plasmid DNA or siRNA constructs), or even proteins such as antibodies, may be transferred or transfected.

Transfection can be verified by any appropriate method, for example quantification of the pDNA delivery to the nucleus by real-time PCR or by measuring the expression of said gene or by measuring the concentration of the expressed protein or in the case of siRNA measurement of the expression of the targeted protein. Suitable methods of transfection, measuring the expression of said gene or measuring the concentration of the expressed protein, or methods for detecting the viability of the cell (such as MTT assays) are well known to the person skilled in the art (see, e.g., Cell Biology. A Laboratory Handbook: vol. 4, Kai Simons, J. Victor Small, Tony Hunter, Julio E. Celis, Nigel Carter, (2005) Elsevier Ltd, Oxford; Auflage: 3rd ed. Literatur; being incorporated herein by reference). If the polyelectrolyte particle further comprises a nucleic acid, complexes formed by the polyelectrolyte particle and the nucleic acid are also known as "polyplexes".

Generally, such polyplexes can be formed in an aqueous solution under conditions that allow complex formation of the polyelectrolyte particle and the nucleic acid. Optionally, these conditions comprise contacting the polyelectrolyte particle and the nucleic acid in an aquous solution at physiological pH. In chemistry, pH is a measure of the acidity or basicity of an aqueous solution. Measuring the pH is routine to the person skilled in the art. In the context of the present invention, the term "physiological pH" denotes the pH of the cell to be transfected in its natural environment. For instance, for mammalian cells, the physiological pH generall is a slightly basic pH typically between about 7.2 and about 7.4. This value is often referred to as physiological pH in biology and medicine. In the context of the present invention, the conditions where the complex is formed can be readily determined by the skilled person (based on the structural features of the particle according to the invention), and preferably refer to an aqueous solution at physiological pH.

In a specific embodiment of the present invention, said nucleic acid may be a DNA and/or RNA fragment, single or double stranded, linear or circular, natural or synthetic, modified or not (see, e.g., US-A-5525711, US-A-4711955 or EP-A-302175 for modification examples) defining a fragment or a portion of a nucleic acid, without size limitation. It may be, *inter alia,* cDNA, genomic DNA, plasmid DNA, messenger RNA, antisense RNA, siRNA, short hairpin RNA, micro-RNA, ribozymes, transfer RNA, ribosomal RNA, or DNA encoding for such types of RNA. The nucleic acid may also be in the form of a plasmid or linear polynucleotide which contains at least one coding sequence of nucleic acid that can

be transcribed and translated to generate polypeptide, ribozyme, antisense RNA or other molecule of interest upon delivery to a cell. The nucleic acid can also be an oligonucleotide which is to be delivered to the cell, e.g., for antisense RNA, siRNA or ribozyme functions. According to the invention, said nucleic acid can also be formulated with viral proteins, or cationic lipids, or liposomes or cationic polymers. "Polynucleotides" and "nucleic acids" are synonyms with regard to the present invention.

In a specific embodiment, both DNA and RNA molecules can be delivered to cells to trigger or to prevent the formation of a protein of interest which may stay within the cell or which will be secreted from the cell. In a more specific embodiment, plasmid DNA is preferred. If the nucleic acids contain the proper genetic information, they will direct the synthesis of relatively large amounts of the encoded polypeptide. The genetic information necessary for expression by a target cell comprise all the elements required for transcription of said DNA into mRNA and for translation of mRNA into polypeptide. Transcriptional promoters suitable for use in various vertebrate ot invertebrate systems are well known to the skilled person. For example, suitable promoters include viral promoters RSV, MPSV, SV40, CMV or 7.5k, vaccinia promoter, constitutive endogenous promoter (i.e., elongation factor 1 alpha (EF1a) inducible promoters, etc. Nucleic acids can also include intron sequences, targeting sequences, transport sequences, sequences involved in replication or integration. Said sequences have been reported in the literature and can be readily obtained by those skilled in the art. Nucleic acids can also be modified in order to be stabilized with specific components as spermine.

Examples of encoded polypeptides or proteins according to the invention are enzyme, hormone, cytokine, membrane receptor, structural polypeptide, transport polypeptide, adhesine, ligand, transcription factor, transduction factor, replication factor, stabilisation factor, antibody, more especially CFTR, dystrophin, factors VIII or IX, E6 or E7 from HPV, MUC1, BRCA1, interferon beta, interferon gamma , interleukin (IL)2, IL-4, IL-6, IL-7, IL-12, GM-CSF (Granulocyte Macrophage Colony Stimulating Factor), tk gene from Herpes Simplex type 1 virus (HSV-1), p53, VEGF. The polynucleotide can also code for an antibody. In this regard, antibody encompasses whole immunoglobulin of any class, chimeric antibodies and hybrid antibodies with dual or multiple antigen or epitope specificities, and fragments, such as single chain antibody, F(ab)2, Fab', Fab including hybrid fragments and anti-idiotypes (see, e.g., US-A-4,699,880).

For complex formation between the polyelectrolyte particle and the nucleic acid, a certain ratio between the polyelectrolyte particle according to the invention and the nucleic acid to be transferred is required. This ratio is also known as "N/P ratio". Determining the optimal N/P ratio is routine to the person skilled in the art. According to an optional embodiment of the invention, the polymer chains comprise positively charged nitrogen atoms and the N/P ratio is between 5 and 50, and preferably between 5 and 20.

As used herein, the term "cells" includes prokaryotic cells and eukaryotic cells, yeast cells, plant cells, insect cells, human or animal cells, in particular mammalian cells. In particular, cancer cells should be mentioned. As used herein, the term "microbioreactor" denotes disposable, parallel-operated polymer based devices with small volumes of cultures which have been proposed as a solution for high-throughput bioprocessing (see, e.g., Zhiyu Zhang et al. (2007), JALA 12:143-51).

It is understood that the step of "contacting the polyelectrolyte particle and a eukaryotic cell or a microbioreactor, comprising the intracellular molecule, under conditions for interaction of said polyelectrolyte particle with the intracellular molecule to form a conjugate" further means to facilitate formation of a conjugate between the polyelectrolyte particle and the intracellular molecule having affinity to the polyelectrolyte particle. In this context, the term "affinity" means that the intracellular molecule can reversibly or irreversibly bind to the polyelectrolyte particle. In this context, the term "specific affinity" means that the intracellular molecule can reversibly or irreversibly bind to the polyelectrolyte particle and that said binding is not accidently. As used herein, the term "conjugate" or "complex" means covalent or non-covalent aggregation of at least two (macro-) molecules.

The inventive method further comprises the step of "recovering the conjugate from the eukaryotic cell or the microbioreactor". This can be accomplished, *inter alia,* by "pull-down" or by "pull-out". Accordingly, the step of "recovering the conjugate from the eukaryotic cell" can comprise releasing intracellular molecules from inside the eukaryotic cell by disruption of the eukaryotic cell (i.e. cell lysis), transferring the intracellular molecules on a column placed in a magnetic field and isolating the conjugate from the intracellular molecules (i.e. "pull-down"). As used herein, the term "cell lysis" refers to the breaking down of a cell, often by viral, enzymatic, mechanical (i.e., Dounce homogenizer) or osmotic mechanisms that compromise its integrity. The process of cell lysis is well known to the person skilled in the art. The column used in this method may be a MACS® column or any other device that facilitates placing the cell lysate in a magnetic field in order to isolate the magnetic conjugates from non-magnetic molecules contained in the lysate. An exemplary process scheme is depicted in Table 3 and in Figure 10. Optionally, the method can comprise further washing steps.

Similarly, the step of "recovering the conjugate from the microbioreactor" can comprise transferring the intracellular molecules on a column placed in a magnetic field and isolating the conjugate from the intracellular molecules (i.e. "pull-down"). The column used in this method may be a MACS® column or any other device that facilitates placing the content of the microbioreactor in a magnetic field in order to isolate the magnetic conjugates from non-magnetic molecules contained in the microbioreactor. Suitable reaction conditions are known to the person skilled in the art.

Alternatively, the step of "recovering the conjugate from the eukaryotic cell" can comprise releasing the conjugate from inside the eukaryotic cell by applying a magnetic field to the eukaryotic cell (i.e. "pull-out"). Similarly, the step of "recovering the conjugate from the microbioreactor" can comprise releasing the conjugate from inside the microbioreactor by applying a magnetic field to the microbioreactor.

It is known in the art that "magnetofection" is a simple and highly efficient transfection method that uses magnetic fields to concentrate particles containing nucleic acid into the target cells. This method attempts to unite the advantages of the popular biochemical (cationic lipids or polymers) and physical (electroporation, gene gun) transfection methods in one system while excluding their inconveniences (low efficiency, toxicity). The magnetofection principle is to associate nucleic acids with cationic magnetic nanoparticles: these molecular complexes are then concentrated and transported into cells supported by an appropriate magnetic field. In this way, the magnetic force allows a very rapid concentration of the entire applied vector dose onto cells, so that 100% of the cells get in contact with a significant vector dose. Magnetofection has been adapted to all types of nucleic acids (DNA, siRNA, dsRNA, shRNA, mRNA, ...), non viral transfection systems (transfection reagents) and viruses. It has been successfully tested on a broad range of cell lines, hard-to-transfect and primary cells. Suitable reaction conditions to perform a magnetfection are known to the person skilled in the art. Without wishing to be bound by theory, it is thus believed that the same principle can be used to recover the magnetic conjugate from the eukaryotic cell or the microbioreactor. Without wishing to be bound by theory, it is further believed that this pull-out method has the additional advantage that at least some of the eukaryotic cells are not substantially damaged.

It is understood that the step of "isolating the intracellular molecule from the conjugate" means to isolate the intracellular molecules which are bound to the polyelectrolyte particle. This may be accomplished by a suitable displacement reaction, wherein the polymer chains comprising the intracellular molecule are displaced from the magnetic nanoparticle, e.g. by addition of a suitable polyanion For instance, the conjugates can be incubated with poly(methacrylic acid) in order to release the molecules bound to the polyelectrolyte particle as complexes between the polyelectrolyte particle and PMAA are built (in the following also designated as "complex II"). These newly built complexes II can be further separated from the released molecules, e.g., using the MACS® system as previously described for the conjugates isolation step (see above). Suitable reaction conditions are known to the person skilled in the art.

Optionally, the intracellular molecules obtained in the inventive method may subsequently be analyzed. The analysis of the intracellular molecules may be carried out via any suitable detection technique known in the art. Suitable detection techniques are known to the person skilled in the art. In a specific embodiment of the present invention, the analysis of the intracellular molecule is accomplished, for example, by the separation of proteins on a polyacrylamide gel, followed by visualization of the proteins. Alternatively, the intracellular molecule can be detected in two-dimensional gel electrophoresis systems. Two-dimensional gel electrophoresis is well known in the art and typically involves isoelectric focusing along a first dimension followed by SDS-PAGE electrophoresis along a second dimension. The analysis of 2D SDS-PAGE gels can be performed by determining the intensity of protein spots on the gel (e.g. as described above), or can be performed using immune detection. In other embodiments, the intracellular molecule is analyzed by mass spectroscopy.

Immunological tests which may be used in the context of the present invention, in particular for the detection purposes of the present invention, include, for example, competitive and non-competitive assay systems using techniques such as western blots, radioimmunoassay like RIA (radio-linked immunoassay), ELISA (enzyme linked immunosorbent assay), "sandwich" immunoassays, immunoprecipitation assays, precipitation reactions, gel diffusion precipitin reactions, immunodiffusion assays, agglutination assays, e.g. latex agglutination, complement-fixation assays, immunoradiometric assays, fluorescent immunoassays, e.g. FIA (fluorescence-linked immunoassay), chemiluminescence immunoassays, electrochemiluminescence immunoassay (ECLIA) and protein A immunoassays. Such assays are routine and well known to the person skilled in the art.

Suitable visualization methods to detect the intracellular molecule are known to the person skilled in the art. Typical methods to be used comprise fluorometric, luminometric and/or enzymatic techniques. Fluorescence is normally detected and/or quantified by exposing fluorescent labels to light of a specific wavelength and thereafter detecting and/or quantifying the emitted light of a specific wavelength. The presence of a luminescently tagged affinity ligand may be detected and/or quantified by luminescence developed during a chemical reaction. Detection of an enzymatic reaction is due to a color shift in the sample arising from chemical reaction.

If intracellular molecules are proteins, an isolated intracellular molecule can be optionally characterized by mass spectrometric (MS) techniques (e.g., by in-gel digestion of target proteins or by standard on-bead digestion of probe-labeled target proteins after affinity purification). In particular, the sequence of an isolated intracellular molecule can be determined using tandem mass spectrometric techniques, and by application of sequence database searching techniques, the intracellular molecule from which a sequenced peptide originated can be identified.

In a preferred embodiment of the present invention, the method further comprises analyzing the intracellular molecule by one or more means selected from the group consisting of liquid chromatography, electrophoretic separation, precipitation, immunocapture, microfluidic separation and mass spectrometry.

"Chromatography" is the collective term for a set of laboratory techniques for the separation of mixtures. It involves passing a mixture dissolved in a "mobile phase" through a stationary phase, which separates the proteins of interest from other proteins in the mixture based on differential partitioning between the mobile and stationary phases. In the context of the present invention, liquid chromatography may be preparative or analytical. The purpose of preparative chromatography is to separate the components of a mixture for further use (and is thus a form of purification). Analytical chromatography is done normally with smaller amounts of material and is for measuring the relative proportions of analytes in a mixture. The two techniques are not mutually exclusive.

"Electrophoretic separation" (or gel electrophoresis) is a technique used for the separation of protein molecules using an electric field applied to a gel matrix. Electrophoretic separation is usually performed for analytical purposes, but may be used as a preparative technique prior to use of other methods such as mass spectrometry for further characterization.

"Precipitation" is widely used in downstream processing of biological products, such as proteins. This operation serves to concentrate and fractionate the target protein from various contaminants. The underlying mechanism of precipitation is to alter the solvation potential of the solvent and thus lower the solubility of the solute by addition of a reagent.

In the context of the present invention, the term "immunocapture" denotes the use of capture antibodies which are able to isolate target proteins (e.g., large enzyme complexes in their fully intact, fully active states) from protein samples.

"Microfluidic separation" involves the use of microfluidic chip suitable for high efficiency continuous separation of liquid protein samples.

"Mass spectrometry" (MS) is an analytical technique for the determination of the elemental composition of a sample or molecule. It is also used for elucidating the chemical structures of molecules, such as peptides and other chemical compounds. The MS principle consists of ionizing chemical compounds to generate charged molecules or molecule fragments and measurement of their mass-to-charge ratios.

According to a very specific embodiment of the invention, the polyelectrolyte particle further comprises a nucleic acid coding for a target protein, and the method comprises:

(a) contacting said polyelectrolyte particle and a eukaryotic cell comprising the intracellular molecule under conditions for interaction of said polyelectrolyte particle with the intracellular molecule to form a conjugate;
(b) recovering the conjugate from the eukaryotic cell;
(c) isolating the intracellular molecule from the conjugate;
(d) optionally, characterizing and/or identifying the intracellular molecule;
(e) contacting the polyelectrolyte particle and a further eukaryotic cell under the conditions according to (a), wherein the further eukaryotic cell comprises a different amount of the intracellular molecule compared to the eukaryotic cell according to (a);
(f) comparing the amounts of target protein in the eukaryotic cell and in the further eukaryotic cell; and
(g) optionally, categorizing the intracellular molecule with regard to its capability to modify a transfection reaction.

It is understood that the step of "characterizing and/or identifying the intracellular molecule" means to analyze the intracellular molecule using any of the detections methods described above.

Furthermore, the step of "contacting the polyelectrolyte particle and a further eukaryotic cell under the conditions according to (a), wherein the further eukaryotic cell comprises a different amount of the intracellular molecule compared to the eukaryotic cell according to (a)" is to be understood to mean the transfection of a further population of eukaryotic cells which compared to the population of eukaryotic cells used in (a) have an altered amount of the intracellular molecule. If the intracellular molecule is a protein, this step is to be understood to mean that both populations of eukaryotic cells have different expression profiles of the protein. For example, in the further population of eukaryotic cells, the protein expression of the intracellular molecule may be altered (e.g., by knock-out or overexpression of the protein). Suitable methods are known to the skilled person.

It is understood that the step of "categorizing the intracellular molecule with regard to its capability to modify a transfection reaction" means to identify whether and/or how the intracellular molecule might be used to modify a transfection reaction, in particular to further improve the reaction with regard to efficiency and/or cytotoxicity of the used polyelectrolyte particle.

Accordingly, in a further embodiment, the present invention relates to the use of an intracellular molecule as obtained in the inventive method for modifying the expression of a nucleic acid transfected into a eukaryotic cell by means of a polyelectrolyte particle as defined herein above. For example, the expression of the intracellular molecule (if being a protein) may be altered (e.g., by knock-out or overexpression of the protein). Suitable methods are known to the skilled person.

In a further embodiment, the present invention relates to the use of a polyelectrolyte particle as defined herein above for identifying and/or isolating a subset of eukaryotic cells comprising the polyelectrolyte particle from a plurality

of eukaryotic cells, the use comprising contacting the polyelectrolyte particle and the plurality of eukaryotic cells under conditions to allow transfer of the polyelectrolyte particle into the eukaryotic cells, and identifying and/or isolating the subset of eukaryotic cells having magnetic properties.

In particular, it has been surprisingly found that eukaryotic cells comprising the polyelectrolyte particle which is in complex with a nucleic acid can be separated from eukaryotic cells not comprising the complex by utilizing the magnetic properties of the eukaryotic cells comprising the complex. These "magnetic cells" can be separated from the "non-magnetic cells", e.g., using the MACS® system as previously described for the conjugates isolation step (see above). Suitable reaction conditions are known to the person skilled in the art. Without wishing to be bound, it is believed that this can lead to an enrichment of eukaryotic cells comprising the polyelectrolyte particle / nucleic acid complex and can further improve the efficiency of the transfection reaction (by sorting out eukaryotic cells not comprising the complex).

In a further embodiment, the present invention relates to the use of a polyelectrolyte particle as defined herein above for isolating a complex formed between the polyelectrolyte particle and a nucleic acid from polyelectrolyte particles not in complex with the nucleic acid.

This means that the magnetic property of the polyelectrolyte particle as defined herein above can also be utilized to separate free particles from polyplexes (i.e. the polyelectrolyte particle / nucleic acid complex). Polyplexes can be separated from free polyelectrolyte particles using size exclusion chromatography, and, unexpectedly, polyplexes depleted from the free polymer are less toxic to the cells (i.e., higher transfection efficiency after removal of the free polymer) (see Example 4). Without wishing to be bound by any theory, it is believed that, despite of their superparamagnetic properties in solution, the polyelectrolyte particle as defined herein above cannot be separated with a magnet. It is further believed that this is due to repulsive electrostatic interactions as well as high solubility of the nanoparticles (due to long soluble polymer chains), and that a separation with a magnetic field is only feasible after aggregation (i.e., interaction with a nucleic acid). Since it is very difficult and time-consuming to separate polyplexes from free polyelectrolyte particles using size exclusion chromatography, the separation with a magnetic field offers clear advantages over conventional separation techniques. Without wishing to be bound, it is believed that this can further improve the efficiency of the transfection reaction (by sorting out free polyelectrolyte particle from polyplexes and performing the transfection reaction only with polyplexes).

While the above invention has been described with respect to some of its embodiments, this is in no way to limit the scope of the invention. The person skilled in the art is clearly aware of further embodiments and alterations to the previously described embodiments that are still within the scope of the present invention.

## EXAMPLES

### Comparative Example 1: "Si-PDMAEMA"

A well defined homopolymer with a star-shaped topology (20 arms) was synthesized with the help of atom transfer radical polymerization from a silsesquioxane initiator. For a typical polymerization procedure of the particle "Si-PDMAEMA", a silsesquioxane initiator for atom transfer radical polymerization is used, as published e.g. by H. Mori et al. (2003), Journal of the American Chemical Society 125: 3712-3713; H. Mori et al. (2004), Macromolecules 37: 5228-5238; and S. Muthukrishnan et al. (2005), Macromolecules 38: 10631-10642; which publications are incorporated herein by reference. The synthesis of star-shaped Si-PDMAEMA particles was published by F. Plamper et al. (2007), Macromolecules 40: 5689, which publication is incorporated herein by reference. The method disclosed therein can also be used to synthesize further star-shaped Si-PDMAEMA particles.

### Example 2: "NP@PDMAEMA"

Well defined homopolymers with a star-shaped topology were synthesized with the help of atom transfer radical polymerization from iron oxide nanoparticles.

Materials

AVS Titrinorm buffer solution pH 10 (VWR), 2-bromoisobutyryl bromide (98 %, Sigma-Aldrich), (2-dimethylamino)ethyl methacrylate (99 %, Sigma-Aldrich), di(ethylene glycol) methyl ether methacrylate (95 %, Sigma-Aldrich), dioctyl ether (99 %, Sigma-Aldrich), 1,1,4,7,10,10-hexamethyltriethylenetetramine (97 %, Sigma-Aldrich), 3-hydroxytyramine hydrochloride (98 %, Sigma-Aldrich), Iron(0)pentacarbonyl (99.9 %, Sigma-Aldrich), oleic acid (90 %, Fluka), sodium carbonate monohydrate (99.5%, Sigma-Aldrich) and sodium tetraborate decahydrate (99.5 %, Sigma-Aldrich) were purchased and used as received. Copper (I) chloride was purified according to FA Plamper et al. (2007), Macromolecules 40, 5689-5697, and the stabilizer of the monomers were removed via a column of basic aluminium oxide. For dialysis a tube from regenerated cellulose (ZelluTrans, Roth) with a MWCO of 6-8 kDa was used.

Cell culture materials, media and solutions were from PAA Laboratories (Cölbe, Germany). Serum reduced medium OptiMEM was from Invitrogen (Darmstadt, Germany). Plasmid DNA was prepared by using the EndoFree Plasmid Kit from Qiagen (Hilden, Germany). Ultrapure Milli-Q water was used for the preparation of all aqueous solutions and for dialysis.

Plasmid pEGFP-N1 (4.7 kb, Clontech, USA) encoding EGFP driven by the cytomegalovirus immediate early promoter was used in all transfection experiments. The plasmid was amplified in *E. coli DH5 alpha* strain in LB medium to sufficient quantities by using standard molecular biology techniques, including harvesting and purification via Qiagen's Giga-Prep kits. Plasmid DNA (pDNA) concentration and quality were determined by $A_{260}/_{280}$ ratio and by agarose gel electrophoresis.

Synthesis and functionalization of iron oxide nanoparticles

A catechol-terminated initiator was synthesized following the method reported by X. Fan et al. (2005), J. Am. Chem. Soc. 127, 15843-15847. The iron oxide nanoparticles (NP) were synthesized following the strategy disclosed in T Hyeon et al. (2001), J. Am. Chem. Soc. 123, 12798-12801. The iron oxide nanoparticles (500 mg) were functionalized with 250 mg dopamine-initiator in 30 mL THF. The mixture was shaken for 3 days at room temperature. Subsequently, the particles were precipitated with methanol to remove the excess of non-bound initiator and dialyzed further for 4 days in anisole for solvent exchange.

Synthesis of NP@PDMAEMA via "grafting from"-approach

A 250 mL screw cap glass equipped with a septum was charged with 500 mg nanoparticles dispersed in 150 mL anisole, 100 mL DMAEMA (93.3 g, 594 mmol) and 82 mg CuCl (0.83 mmol). The mixture was purged with nitrogen for 30 min before adding 0.23 mL degassed HMTETA (0.19 g, 0.83 mmol) dissolved in 2 mL anisole. The reaction mixture was heated to 60 °C for 42 h. After cooling down to room temperature the reaction was terminated by exposing the mixture to air while stirring for 10 min. The crude product was purified by passing it through a silica column for removing the catalyst and subsequently, precipitated in n-hexane followed by redispersed in 1,4-dioxane and freeze-drying. Final purification was performed by ultrafiltration and controlled temperature induced precipitation.

Ultrafiltration: 200 mg of the crude product was dispersed in deionized water and filtrated with deionized water for one week utilizing a 50 nm MF-Millipore™ membrane filter. The purified particles were stored in deionized water.

Temperature induced precipitation: 200 mg of the grafted particles were dispersed in 20 mL of a boric acid based buffer solution (pH 10). This solution was heated until the particles started to precipitate. The supernatant was decanted and the particles were again dispersed in 20 mL of fresh buffer solution. This temperature induced controlled precipitation was performed 3 times until the particles were finally dispersed and dialysed against deionized water and stored in aqueous solution.For characterizing the grafted polymer, NP@PDMAEMA particles were dissolved in 3 M hydrochloric acid which causes hydrolysis of the iron oxide cores of the hybrid material. Subsequently, the resulting yellow solution was dialyzed for 3 days against deionized water to neutral pH.

The remaining polymer chains were characterized by DMAc-SEC using a PDMAEAMA calibration ($M_n$(PD-MAEMA) = 93000 g/mol; PDI = 1.22). Size exclusion chromatography (SEC) measurements were performed on a system based on GRAM columns (5 μm particle diameter) with $10^2$ and $10^3$ Å pore diameter from PSS equipped with a RI- and UV-detector. SEC was measured at an elution rate of 1 mL/min with N,N-dimethylacetamid (DMAc) as solvent.

A scheme of the synthesis strategy and dual-responsive behavior of NP@PDMAEMA particles (see below) is shown in Figure 1.

Synthesis of NP@PDMAEMA-b-PDEGMA

A glass vessel equipped with a rubber septum and a NIR probe was charged with 1 g of NP@PDMAEMA raw product dispersed in 30 mL Acteonitrile, 2 mL DEGMA (2.04 g, 10.8 mmol) and 10 mg CuCl (0.1 mmol). The mixture was purged with nitrogen for 30 min before adding 0.05 mL degassed HMTETA (46.5 mg, 0.1 mmol) dissolved in 2 mL acetonitrile. The reaction mixture was heated to 60 °C for 19.3 h and terminated after 25.5 % converstion (resulting composition of the copolymer: $PDMAEMA_{590}$-b-$PDEGNIA_{280}$) by cooling down to room temperature and exposing the mixture to air while stirring for 10 min. The crude product was purified by passing it through a silica column for removing the catalyst and subsequently, precipitated in n-hexane followed by redispersing in 1,4-dioxane and freeze-drying. Final purification was performed by ultrafiltration.

Ultrafiltration: 900 mg of the crude product was dispersed in deionized water and filtrated with deionized water for three weeks utilizing a 50 nm MF-Millipore™ membrane filter. The purified particles were stored in deionized water.

Characterization of the NP@PDMAEMA particle

The resulting particle (NP@PDMAEMA) was fully characterized by standard techniques for polymer characterization:

*a) TEM and DLS*

Transmission electron microscopy (TEM) images were taken with a Zeiss CEM902 (EFTEM) electron microscope operated at 80 kV or a Zeiss EM922 OMEGA (EFTEM) electron microscope operated at 200 kV. Samples were prepared through deposition of a drop of the solution onto carbon-coated TEM copper-grids. Afterwards the remaining solvent was removed with a filter paper. Dynamic light scattering (DLS) were carried out by using an ALV DLS/SLS-SP 5022F goniometer with an ALV 5000/E correlator and a He-Ne laser with a wavelength of 632.8 nm. The measurement was performed at a scattering angle of 90° and CONTIN algorithm was applied to analyze the obtained correlation functions. Before measuring, the sample (c = 0.5 g/L) was filtrated through a 0.2 $\mu$ PTFE-filter.

The iron oxide nanoparticles were synthesized via thermal decomposition showing a well defined and rather monodisperse round shape as demonstrated by TEM and DLS (see Figures 2A and 2A). The size of the particles resulted from an evaluation of the TEM-micrographs indicating an average diameter of 9.9 $\pm$ 1.8 nm (average over 300 particles) and 13.2 nm by DLS, respectively. Due to the high contrast of the NP's oleic acid is invisible in the TEM-micrographs but was taken into account by determining the $R_h$ by DLS, which results in a higher value for the diameter.

*b) EDX*

Energy dispersive X-ray Spectroscopy (EDX) analysis was performed on a Leo 1530 Gemini instrument equipped with a field emission cathode and a X-ray detector. The used acceleration voltage was between 0.5 kV and 3.0 kV. For preparation the purified sample was drop-coated and dried onto a silicon wafer.

Energy dispersive X-ray spectroscopy (EDX) is a suitable method to indicate the existence of the bromine end-groups of the attached dopamine based ATRP initiator after the functionalization (see Figure 3A). The evidence of bromine with a characteristic signal at around 1.5 keV is a result of the surface-bound initiator. Because of the high carbon and oxygen content and hence, a high signal strength for both elements the signal for nitrogen with its signal at values < 0.5 keV is completely covered.

*c) FT-IR*

Fourier transform infrared spectroscopy (FT-IR) was carried out on a Spectrum 100 FT-IR spectrometer (Perkin Elmer) using an U-ATR unit. The dried samples were directly placed on top of the U-ATR unit and the measurement was started.

NMR-spectroscopy: $^1$H and $^{13}$C NMR spectra were recorded on a Bruker 300 AC spectrometer using MeOD or $CDCl_3$ (Deutero) as solvent and the calibration was done with the help of the internal solvent signal.

The comparison of the FT-IR spectra of the functionalized NPs (NP@Dopamine initiator) with the spectra of oleic acid stabilized NP's (NP@Oleic acid), pure oleic acid and pure dopamine initiator shows clearly a successful functionalization (Fig. 3B). In case of NP@Oleic acid, the characteristic stretching vibrations of the aliphatic $CH_2$ and $CH_3$ groups at 2900-2800 $cm^{-1}$ and carbonyl groups (C=O) at 1700 $cm^{-1}$ of the oleic acid are observed. After functionalization NP@Dopamine initiator exhibit a broad band at 3700-3100 $cm^{-1}$ as well as a band at 1650 $cm^{-1}$ which correspond to hydroxyl (-OH) and amide (N-H; C=O) bonds, respectively. It is noted that the bands of the molecules close to the surface of the NPs and especially for the binding groups are less pronounced and slightly shifted compared to the pure substances.

*d) TGA*

Thermogravimetric analysis (TGA) was carried out using a Mettler Toledo TGA/SDTA 85 at a heating rate of 10 K/min between 30 and 1000 °C under an air-flow of 60 mL/min. The typical sample size was between 8 mg and 15 mg.. For determining the grafting densities ($\delta_{graft}$) the weight loss given by TGA is used to calculate the amount of molecules per $nm^2$ according to following equation:

$$\delta_{graft} = [(\Delta m / M) \text{ x } N_A] / [Q \text{ x } A_{NP}],$$

with $\Delta m$, M, $N_A$, Q and $A_{NP}$ corresponding to the weight of the grafted molecules or polymer determined by TGA, molecular weight of the dopamine initiator or $M_n$ of a polymer, Avogadro's number, amount of NPs utilized in the TGA measurement (i. e. Q = (initial sample weight - weight loss) divided by the mass of a single nanoparticle assessed by theoretical calculation) and the surface of the NPs. For the calculation the NP's were assumed to be monodisperse with a spherical shape and a diameter of 9.9 nm evaluated by TEM-micrographs, leading to the volume ($V_{NP}$ = 508.1 $nm^3$) and the surface ($A_{NP}$ = 307.9 $nm^2$) of a single iron oxide nanoparticle. The weight per particle ($m_{NP} = 2.5 \cdot 10^{-18}$ g) is obtained by considering the density of maghemite.

TGA measurements of the pure dopamine initiator showed a complete degradation at much higher temperatures than oleic acid. A characteristic degradation step of the dopamine initiator at temperatures above 440 ˚C is observed where approx. 40 % of the initiator mass is degraded. This significant degradation step can also be observed by comparing the TGA curves of NP@Oleic acid and NP@Dopamine initiator (Fig. 4A and 4B).

TGA after the polymerization of DMAEMA showed a weight loss of 73.6 % for the particles purified by controlled precipitation and 90.2 % for NP@PDMAEMA purified by ultrafiltration which is much higher compared to the NP's just carrying the ATRP initiator (Fig. 5). By utilizing the equation and considerations for calculating the grafting density mentioned in the experimental part, the resulting grafting density for the PDMAEMA chains anchored on the surface of the NP's is 46 chains/particle (0.15 chains/$nm^2$) obtained by purification via precipitation method (NP@$PDMAEMA_{46}$) and 160 chains/particle (0.52 chains/$nm^2$) by ultrafiltration (NP@$PDMAEMA_{160}$), respectively.

*e) Stability and magnetic properties*

The polymer grafted particles showed excellent stability and solubility in aqueous media without agglomeration. The TEM-micrograph prepared by drop-coating from an aqueous solution (c = 5 g/L) on a hydrophilized copper grid (Fig. 6) demonstrated clearly well dispersed NP@$PDMAEMA_{160}$ particles. Due to electrostatic repulsive interactions and steric hindrance of the long PDMAEMA chains preventing agglomeration it is impossible to separate the grafted NP's just by applying a magnet. Even by using NdFeB-magnets, which are very powerful, the NP@PDMAEMA particles stay excellently dispersed in solution. Consequently, for collecting the particles by applying a magnet they have at first to be precipitated e.g. heating above the LCST (Fig. 7).

*f) VSM*

Vibrating sample magnetometry (VSM): Magnetization curves at room temperature were recorded with an Lake Shore Vibrating Sample Magnetometer Model 7404 applying field strengths up to 1.4 T. Samples were measured in sealed Kel-F vessels, placed on a fiber glass sample holder between two poles of an electromagnet, and vibrated at a frequency of 82 Hz.

Magnetization measurements, carried out via VSM, proved the superparamagnetic behavior of the unmodified particles and NP@PDMAEMA (Fig. 8). The normalized graphs of both, pure and grafted particles show the typical symmetrical sigmoidal shape without hysteresis which means, that magnetic redistribution takes place via internal (Néel) relaxation. No significant difference between the two samples was observed. These curves are additional fitted with the more complex (compared to the Langevin function) Ivanov model (AO Ivanov et al. (2001), Phys. Rev. E: Stat., Nonlinear, Soft Matter Phys.64, 041405), which assumes a gamma-distribution of the particle size and takes into account an approximated particle interaction. The measurements fit well with the theoretical assumption, which leads to the conclusion (however without wishing to be bound by any theory) that the superparamagnetic behavior of the particles is remaining and not significantly influenced by the grafting-from approach.

*g) Thermoresponsiveness*

Turbidity measurements for determining the cloud points were performed by using a titrator (Titrando 809, Metrohm, Herisau, Switzerland) equipped with a turbidity probe ($\lambda_0$ = 523 nm, Spectrosense, Metrohm) and a temperature sensor (Pt 1000, Metrohm). The temperature program (1 K/min) was run by a thermostat (LAUDA RE 306 and Wintherm_ Plus software), using a home-made thermostatable vessel. The cloud points were determined from the intersection of the two tangents applied to the two linear regimes of the transmission curve at the onset of turbidity.

Due to the reversible binding we used freshly purified particles bearing 46 arms (NP@$PDMAEMA_{46}$) determined by TGA for turbidity measurements determining the dual-responsive behavior (Fig. 9A). The coil-globule transitions at the cloud point of the grafted particles are sharp and strongly dependent on pH. At pH 7, the PDMAEMA chains are highly positively charged due to protonation (pKa $\approx$ 8), which results in an improved solubility in water, as well as in a cloud point at relatively high values (80.1 ˚C). By increasing the pH the PDMAEMA becomes progressively less charged. Consequently, the solubility in water decreases which causes a shift of the cloud point to lower temperatures.

Beside NP@$PDMAEMA_{46}$ the linear cleaved PDMAEMA chains were also analyzed via turbidity measurements. Since, there is a strong influence of the molecular weight on the LCST especially at high pH the LCST behavior for the grafted PDMAEMA chains attached on the surface of the NP's are supposed to be altered with respect to the cleaved chains. By increasing the molecular weight the LCST of PDMAEMA is shifted to lower values at high pH independent of the architecture of the polymer (FA Plamper et al. (2007), Macromolecules 40, 8361-8366). Due to the PDMAEMA single chains sticking closely packed on the particles the LCST behavior of NP@PDMAEMA$_{46}$ resembles the behavior of star shaped PDMAEMA with a very high molecular weight. A direct comparison of the cloud points showed a considerable shift of the LCST between the cleaved PDMAEMA and NP@PDMAEMA$_{46}$ which can be already observed at pH 8 and gets more obvious at pH 9 and 10, respectively (Fig. 9B, Table 1).

**Table 1**: Cloud points [°C] of NP@PDMAEMA$_{46}$, cleaved linear PDMAEMA homopolymer under different conditions (c = 0.1 g/L, heating rate = 1 K/min).

| | pH 7 | pH 8 | pH 9 | pH 10 |
|---|---|---|---|---|
| NP@PDMAEMA$_{46}$ | 80.1 | 47.6 | 32.0 | 27.9 |
| Cleaved PDMAEMA | 79.7 | 49.7 | 36.4 | 33.0 |

As previously mentioned PDMAEMA shows a high solubility at pH 7 and below resulting in identical LCST values for high and low molecular weight PDMAEMA. The difference in the LCST behavior at higher pH confirms the successful grafting of PDMAEMA from the iron oxide nanoparticles

Description of cellular assays

The CHO-K1 (CCL-61, ATCC), A549 (CCL-185, ATCC) and L929 (CCL-1, ATCC) cell lines used in the transfection and cytotoxicity experiments, respectively, were maintained in RPMI 1640 (CHO-K1) and MEM (L929) cell culture media supplemented with 10 - 20 % fetal calf serum (FCS) (as recommended by ATCC), 100 μg/mL streptomycin, 100 IU/mL penicillin, and 2-4 mM L-Glutamine. Cells were cultivated at 37 °C in a humidified 5 % $CO_2$ atmosphere.

The efficiency of the NP@PDMAEMA particles as transfection reagent was explored in the CHO-K1 and A549 cell lines and transfection efficiencies were compared to the gold standard poly(ethylene imine)(b-PEI, 25 kDa). For transfection of the CHO-K1 and A549 cells were incubated at a density of 2x10$^5$ cells/well in 6-well plates overnight prior transfection. One hour prior transfection, cells were rinsed with DPBS and supplemented with OptiMEM. pDNA/polymer polyplexes were prepared by mixing 3 μg pDNA with amounts of the respective polycation stock solution to achieve the suitable N/P ratio in a final volume of 200 μL of aqueous 150 mM NaCl solution. Solutions were vortexed and incubated for several minutes at room temperature to allow polyplexes formation. The polyplexe suspension was added to the cells and the plates were centrifuged for 5 min and placed for 4 h in the incubator. Afterwards, the medium was removed by aspiration, fresh growth medium was added, and the cells were further incubated overnight. For analysis, the cells were harvested by trypsinization and resuspended in DPBS. Dead cells were identified *via* counterstaining with propidium iodide. The relative expression of EGFP fluorescence of 1x10$^4$ cells was quantified *via* flow cytometry using a Cytomics FC 500 (Beckman Coulter, Krefeld, Germany).

Results

The efficiency of this polycation as potential transfection reagent was explored, under standard conditions and its transfection efficiency was compared to the gold standard poly(ethylene imine)(b-PEI, 25 kDa). In the CHO-K1 cell line, the NP@PDMAEMA$_{46}$ improved the transfection efficiency up to almost 5-fold depending on the N/P ratio utilized and performed best at N/P 10 to N/P 20. Averaged transfection efficiencies, at optimal N/P ratio, between 53.5 % and 61.4 % were obtained with the NP@PDMAEMA$_{46}$ polycation. Corresponding experiments with b-PEI led at most to 27.6 % transfected cells. Alternatively, we synthesized the 160 arms variant, and also the 121 arms variant as block copolymer (NP@DEGMA-PDMAEMA$_{121}$). The latter molecule exhibited improved transfection efficiency at lower N/P ratio, almost 3-fold higher than the NP@PDMAEMA$_{160}$.

**Example 3: "Utilization of the NP@PDMAEMA$_{46}$ as pull-down system"**

In a preliminary experiment, it could be shown that cells transfected with pDNA complexed with the NP@PDMAEMA$_{46}$ polycation and incubated in the vicinity of the magnet displayed magnetic properties. Moreover, it could be shown that A549 cells transfected with NP@PDMAEMA$_{46}$ but not with Si-PDMAEMA can be specifically separated using a magnetic field (MACS® system) (Table 2). Interestingly, though the data in Table 2 showed that ~

84% of the cells transfected with NP@PDMAEMA$_{46}$- based polyplexes displayed magnetic properties only a fraction of these cells expressed the transgene 48h after transfection (data not shown). Similar observations were already made with rhodamine-labeled PEI and PDMAEMA polycations (Schallon et al., 2010). Without wishing to be bound by any theory, one hypothesis to explain this phenomenon might be that the tested polycations easily go through the cytoplasmic membrane but not through the nuclear membrane. This important observation is under investigation.

**Table 2**: Magnetic properties of A549 cells transfected with NP@PDMAEMA$_{46}$- or Si-PDMAEMA-based polyplexes.

| | Cell concentration (x $10^6$ cells/ml) | |
|---|---|---|
| Transfection reagent | Si-PDMAEMA | NP@PDMAEMA$_{46}$ |
| Input | 2.3 | 2.5 |
| Flow through | 2.2 | 0.4 |
| Eluat (after magentic field removal) | 0.1 | 2.1 |

So far, the intracellular mechanisms leading to efficient transfection are hardly described at the molecular level. Hence, the newly acquired magnetic property of the cells after transfection with NP@PDMAEMA$_{46}$ polycation might be a useful tool to analyze these intracellular mechanisms in particular to identify intracellular molecules interacting with the polyplexes and playing a role in its transport to the nucleus. Toward this goal, a procedure for isolation of these conjugates (conjugate I) was developed (the various steps of this procedure are summarized in Table 3).

At first, the transfected cells were collected by trypsinization and futher lysed in a Dounce homogenizer. The cell lysate was then transfered into a MACS® column (MACS® System, Miltenyi Biotec) placed a magnet field. The magnetic conjugates I are retained in the column whereas the cellular components which are not bound to the polyplexes run through the column. In the next step, the magnetic field was removed and the conjugates I were collected in a separate tube (Figure 10). Further on in order to facilitate the analysis of the components bound to the magnetic polyplexes, the conjugates I were incubated with poly(methacrylic acid) (PMAA, 67.2 kDa) (2,5 nmol (COOH/P = 200)PMAA 10min, mixing). This step triggered the release of molecules bound to the polycation as complexes between NP@PDMAEMA$_{46}$ and PMAA were built (complex II). These newly built complexes II could be further separated from the released molecules using the MACS® system as previously described for the conjugates I separation (see above). To analyze putative proteins that might be contained in the released molecules fraction, a first analysis using the SDS-PAGE (4 % acrylamide) method was performed (Figure 11).

**Table 3**: Steps for the isolation of intracellular molecules which interact with the NP@PDMAEMA$_{46}$-pDNA polyplexes

| | **Steps** | **Method** | **Analytical methods** |
|---|---|---|---|
| 1 | Collection of the transfected cells | Trypsinization (adherent cells)<br>Centrifugation (suspension cells) | Cell counter<br>(ViCell, Beckmann Coulter)<br>Flow cytometry |
| 2 | Preparation of cell lysate | Dounce homogeniser | SDS-PAGE (Figure 10, lane 2) |
| 3 | Separation of the conjugates I [NP@PDMAEMA$_{46}$-pDNA polyplexes/intracellular molecules] | MACS® system Bound fraction collected | |
| 4 | Incubation of the conjugates I [NP@PDMAEMA$_{46}$-pDNA polyplexes/intracellular molecules] with PMAA | | |
| 5 | Separation of the newly built complexes II [NP@PDMAEMA$_{46}$/PMAA] | MACS® system<br>Flow through and bound fraction collected | SDS-PAGE<br>Flow through<br>(Figure 10, lane 4) After magnetic field removal (Figure 10, lane 3) |

The preliminary results displayed in Figure 11 show that proteins that have bound to polyplexes after transfection could be isolated. There are ongoing efforts to improve, the presented method, in particular reducing the viscosity of the cell lysate (Table 3, step 2). Moreover, washing steps will be introduced.

**Example 4: "Separation of polyplexes from the free polymer prior to transfection"**

The magnetic properties of NP@PDMAEMA can also be utilized to separate the free polymers from the polyplexes. The influence of free polymer during transfection is still not clearly defined for most polycations. In the case of PEI, an excess of free polymer seems to be required for successful transfection outcomes. In that case, destabilization of the plasma membrane as well as the boost of the buffering capacity in the endosomes have been suggested. For PDMAEMA very little is known on the influence of free polymer. It was shown that polyplexes could be separated from free PDMAEMA using size exclusion chromatography, that polyplexes depleted from the free polymer were less toxic to the cells, and that PDMAEMA-based polyplexes, but not PEI-based polyplexes performed better (i.e., higher transfection efficiency) after removal of the free polymer (A. Schallon, PhD in preparation).

Without wishing to be bound by any theory, despite of their superparamagnetic properties in solution, the free NP@PDMAEMA polycations cannot be separated with a strong permanent magnet. This is due to repulsive electrostatic interactions as well as high solubility of the nanoparticles (due to long soluble polymer chains). It was shown that a separation with a magnetic field is only feasible after aggregation (i.e., interaction with plasmid DNA).

**Claims**

1. An *in vitro* method for obtaining an intracellular molecule having specific affinity for a polyelectrolyte particle, wherein the polyelectrolyte particle comprises at least 15 polymer chains, which have a positive net charge and radiate outward from a functionalized magnetic nanoparticle so as to form the polyelectrolyte particle, wherein each polymer chain comprises at least 40 covalently linked monomer units on average, and wherein the method comprises:

   (a) contacting said polyelectrolyte particle and a eukaryotic cell or a microbioreactor, comprising the intracellular molecule, under conditions for interaction of said polyelectrolyte particle with the intracellular molecule to form a conjugate;
   (b) recovering the conjugate from the eukaryotic cell or the microbioreactor; and
   (c) isolating the intracellular molecule from the conjugate.

2. The method according to claim 1, wherein the polymer chains are linear polymer chains and the polyelectrolyte particle does not comprise any branched or cross-linked polymer chains.

3. The method according to claim 1 or 2, wherein the polymer chains are each formed at least in part by the polymerisation of ethylenically unsaturated monomers selected from the group consisting of dialkylamino alkyl(alk)acrylates, dialkylamino alkyl(alk)acrylamides, hydroxy alkyl(alk)acrylates, hydroxy alkyl(alk)acrylamides, alkyl(alk)acrylates and alkyl(alk)acrylamides.

4. The method according to any of the preceding claims, wherein the polymer chains are each formed at least in part by the polymerisation of ethylenically unsaturated monomers comprising monomers having the following general formula (I):

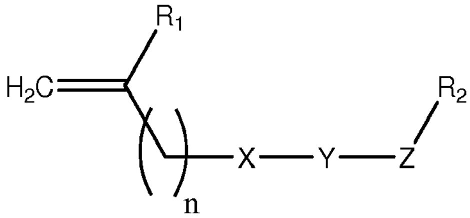

(I)

   wherein:

   $R_1$ is selected from hydrogen or optionally substituted $-(C_1-C_3)$alkyl;
   X is selected from -O-C(O)-, -C(O)-O-, $-NT_3-C(O)-$ or $-C(O)-NT_3-$;

Y is $-(CH_2)_m-$;
Z is selected from -O-, $-(O-CH_2CH_2)_o-$, $-(CH_2CH_2-O)_o-$, -O-C(O)-, -C(O)-O-, $-NT_3-$, - $NT_3-C(O)-$ or $-C(O)-NT_3-$, or is absent;
n is an integer selected from 0, 1, or 2;
m is an integer selected from 0, 1, 2, 3, 4, or 5;
o is an integer selected from 1 to 10;
$R_2$ is selected from -H, or $-(C_1-C_3)$alkyl which is unsubstituted or substituted with 1 or 2 independently selected $R_4$ groups;
each $R_4$ is independently selected from $-OT_3$, $-N(T_1)(T_2)$, $-N(T_3)C(=O)T_3$, or $-N(T_3)C(=O)OT_3$; and
each $T_1$, $T_2$, and $T_3$ is independently selected from -H or $-(C_1-C_3)$alkyl.

**5.** The method according to any of the preceding claims, wherein the polymer chains are each formed at least in part by the polymerisation of ethylenically unsaturated monomers comprising monomers having a general formula selected from the group consisting of formulae (II), (III) and (IV), wherein formulae (II), (III) and (IV) have the following chemical structures:

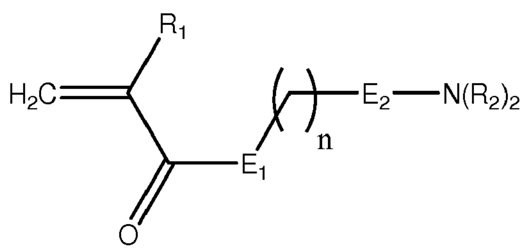
(II),

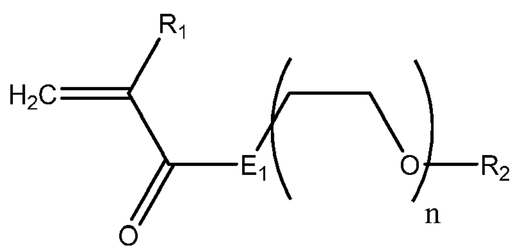
(III),

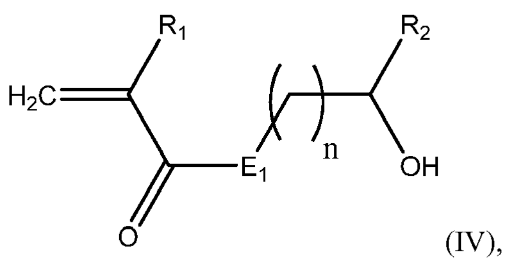
(IV),

wherein:

in formula (II):

$R_1$ is selected from -H, $-CH_3$ or $-CH_2CH_3$;
$E_1$ is selected from -O-, -NH-, or $-N(CH_3)-$;
n is an integer selected from 0, 1 or 2;
$E_2$ is selected from $-(NH-CH_2)_m-$, $-(CH_2-NH)_m-$, $-(NH-CH_2CH_2)_m-$, $-(CH_2CH_2-NH)_m-$, $-(N(CH_3)-CH_2)_m-$, $-(CH_2-N(CH_3))_m-$, $-(N(CH_3)-CH_2CH_2)_m-$, $-(CH_2CH_2-N(CH_3))_m-$, or is absent;
m is an integer selected from 0, 1, 2, 3, 4, 5, or 6; and
$R_2$ is selected from -H, $-CH_3$ and $-CH_2CH_3$;

in formula (III):

$R_1$ is selected from -H, $-CH_3$ or $-CH_2CH_3$;
$E_1$ is selected from -O-, -NH-, or $-N(CH_3)-$;
n is an integer selected from 1, 2, 3, 4, 5, 6, 7, 8, 9, or 10; and
$R_2$ is selected from -H, $-CH_3$ and $-CH_2CH_3$;

in formula (IV):

$R_1$ is selected from -H, $-CH_3$ or $-CH_2CH_3$;
$E_1$ is selected from -O-, -NH-, or $-N(CH_3)-$;
n is an integer selected from 0, 1, 2, or 3; and
$R_2$ is selected from -H, $-CH_3$ and $-CH_2CH_3$-

**6.** The method according to any of the preceding claims, wherein the functionalized magnetic nanoparticle is a functionalized maghemite nanoparticle, optionally a dopamine-functionalized maghemite nanoparticle based core.

**7.** The method according to any of the preceding claims, wherein the polymer chains comprised by the polyelectrolyte particle together have a molecular weight of at least 500 kDa, 550 kDa, 600 kDa, 650 kDa, 700 kDa, 750 kDa, 800 kDa, 850 kDa, 900 kDa, 950 kDa, 1000 kDa, 1500 kDa, 2000 kDa, 2500 kDa, 3000 kDa, 3500 kDa, 4000 kDa, 4500 kDa, 5000 kDa, 6000 kDa, 7000 kDa, 8000 kDa, 9000 kDa, 10,000 kDa, 15,000 kDa, 20,000 kDa, or 30,000 kDa.

**8.** The method according to any of the preceding claims, wherein the polyelectrolyte particle further comprises a nucleic acid, optionally being selected from the group consisting of cDNA, genomic DNA, plasmid DNA, messenger RNA, antisense RNA, siRNA, short hairpin RNA, micro-RNA, ribozymes, transfer RNA, ribosomal RNA, and DNA encoding for such types of RNA.

**9.** The method according to any of the preceding claims, wherein the intracellular molecule is selected from an RNA, a lipid, a carbohydrate, a phospholipid, a peptide, an oligopeptide, a polypeptide, a monomeric protein and a multimeric protein.

**10.** The method according to any of the preceding claims, wherein the intracellular molecule comprises at least one functionality selected from the group consisting of:

(a) binding of the intracellular molecule to a ligand or antigen;
(b) exertion of a catalytic activity, optionally an enzymatic activity, by the intracellular molecule;
(c) susceptibility of the intracellular molecule to a catalytic activity, optionally an enzymatic activity;
(d) facilitation by the intracellular molecule of altered transport of the polyelectrolyte particle across a biological membrane;
(e) facilitation by the intracellular molecule of altered transport of the polyelectrolyte particle in the intracellular compartment;
(f) influence by the intracellular molecule on expression of at least one gene in a population of eukaryotic cells; and
(g) influence by the intracellular molecule on the growth or metabolism of a population of eukaryotic cells.

**11.** The method according to any of the preceding claims, wherein polyelectrolyte particle further comprises a nucleic acid coding for a target protein, and the method comprises:

(a) contacting said polyelectrolyte particle and a eukaryotic cell comprising the intracellular molecule under conditions for interaction of said polyelectrolyte particle with the intracellular molecule to form a conjugate;
(b) recovering the conjugate from the eukaryotic cell;
(c) isolating the intracellular molecule from the conjugate;
(d) optionally, characterizing and/or identifying the intracellular molecule;
(e) contacting the polyelectrolyte particle and a further eukaryotic cell under the conditions according to (a), wherein the further eukaryotic cell comprises a different amount of the intracellular molecule compared to the eukaryotic cell according to (a);
(f) comparing the amounts of target protein in the eukaryotic cell and in the further eukaryotic cell; and
(g) optionally, categorizing the intracellular molecule with regard to its capability to modify a transfection reaction.

**12.** The method according to any of the preceding claims, wherein recovering the conjugate from the eukaryotic cell or the microbioreactor comprises

(a) releasing intracellular molecules from inside the eukaryotic cell by disruption of the eukaryotic cell, transferring the intracellular molecules on a column placed in a magnetic field and isolating the conjugate from the intracellular molecules; or
(b) releasing the conjugate from inside the eukaryotic cell or the microbioreactor by applying a magnetic field to the eukaryotic cell or the microbioreactor.

**13.** Use of a polyelectrolyte particle as defined in any of claims I to 8 for identifying and/or isolating a subset of eukaryotic cells comprising the polyelectrolyte particle from a plurality of eukaryotic cells, the use comprising contacting the polyelectrolyte particle and the plurality of eukaryotic cells under conditions to allow transfer of the polyelectrolyte particle into the eukaryotic cells, and identifying and/or isolating the subset of eukaryotic cells having magnetic properties.

**14.** Use of a polyelectrolyte particle as defined in any of claims 1 to 8 for isolating a complex formed between the polyelectrolyte particle and a nucleic acid from polyelectrolyte particles not in complex with the nucleic acid.

**15.** Use of an intracellular molecule as obtained in the method according to any of claims 1 to 12 for modifying the expression of a nucleic acid transfected into an eukaryotic cell by means of a polyelectrolyte particle as defined in any of claims 1 to 8.

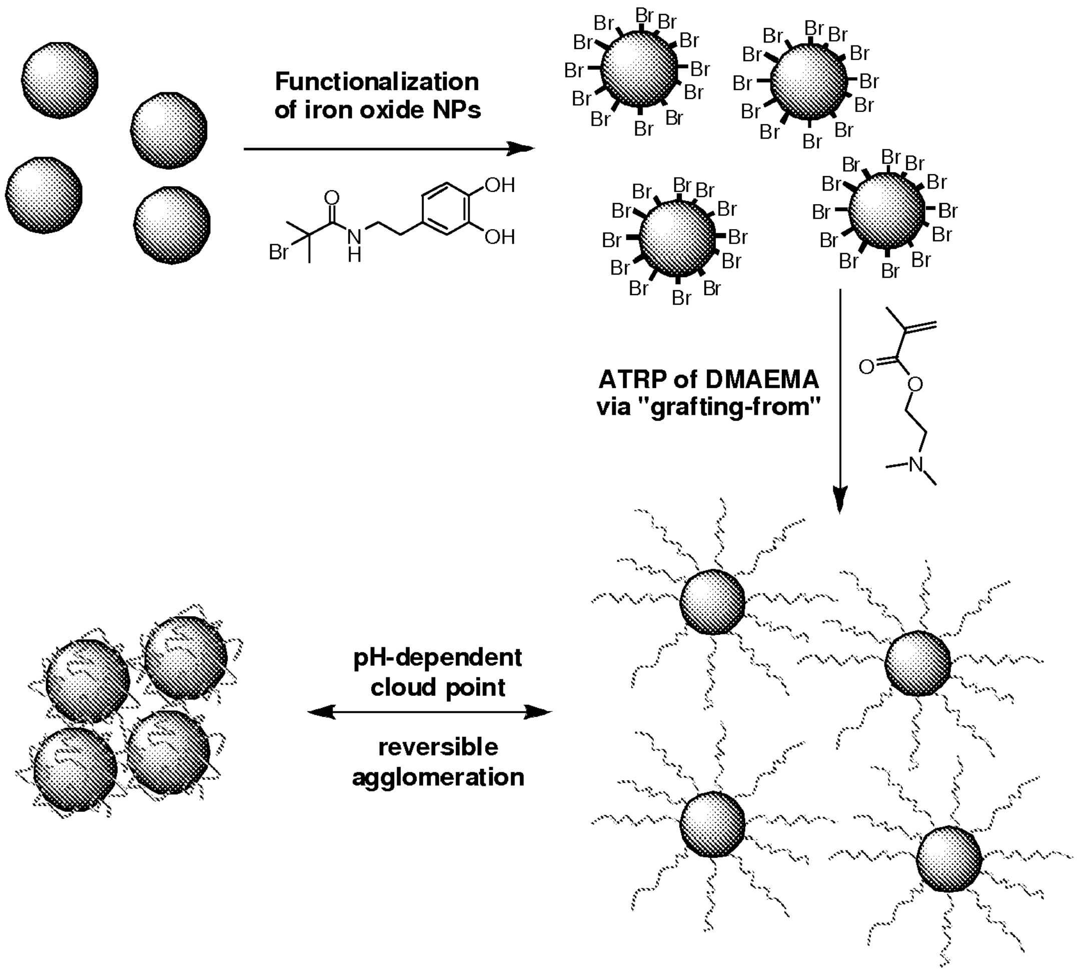
Functionalization
of iron oxide NPs
ATRP of DMAEMA
via "grafting-from"
pH-dependent
cloud point
reversible
agglomeration

Figure 1

A:

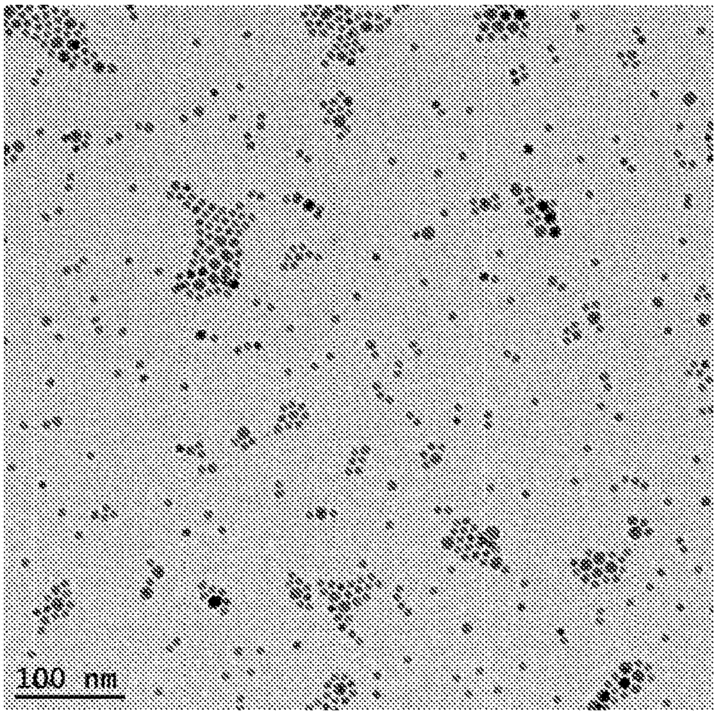
100 nm

B:

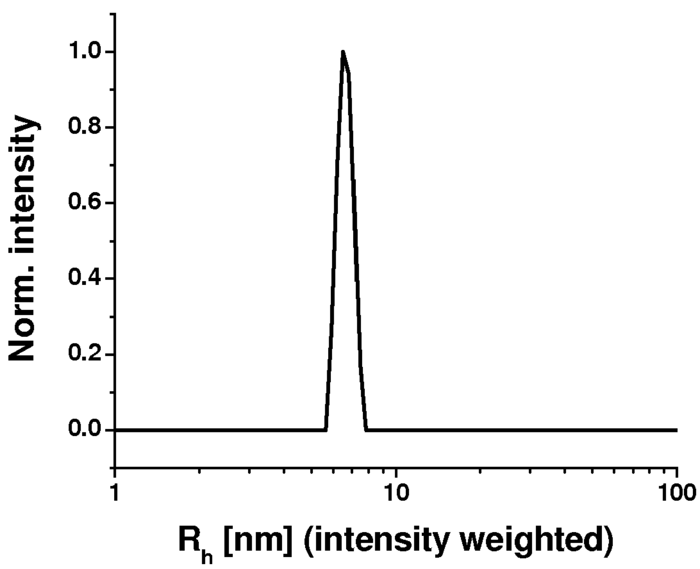
Norm. intensity
1.0
0.8
0.6
0.4
0.2
0.0
1
10
100
R_h [nm] (intensity weighted)

Figure 2

A:

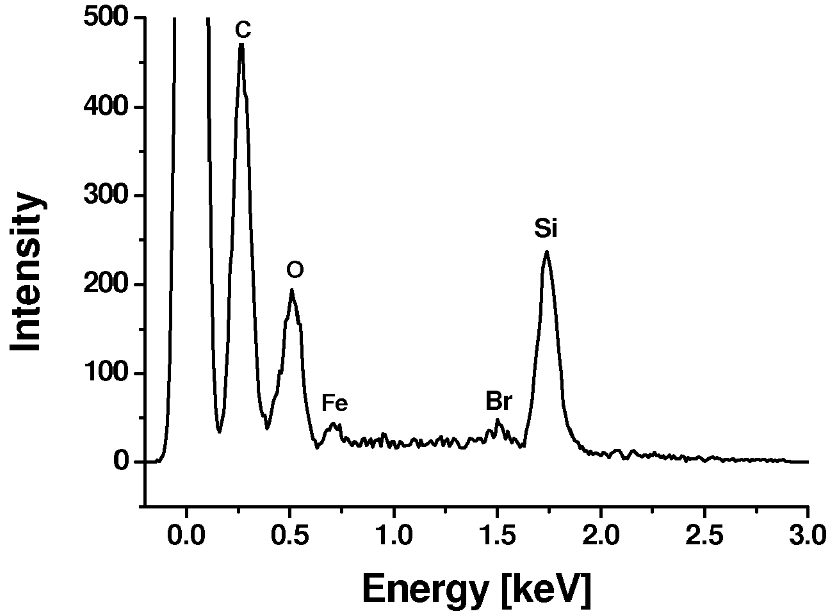

C
O
Si
Fe
Br
Intensity
Energy [keV]


B:

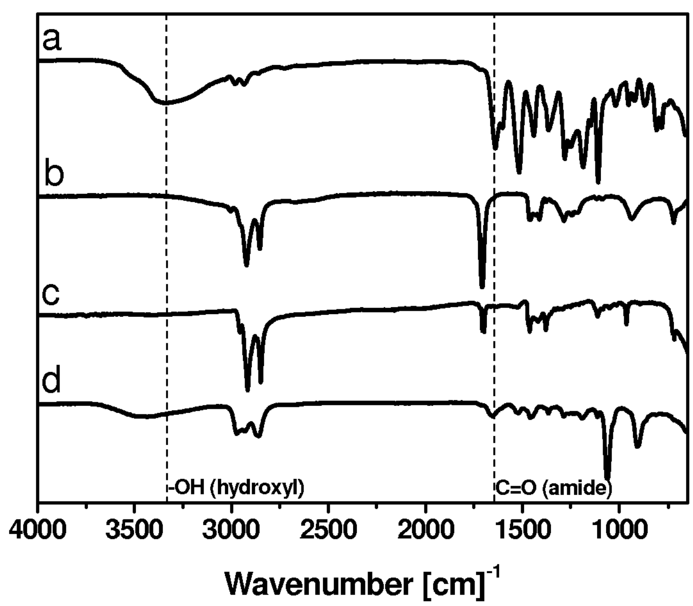

a
b
c
d
-OH (hydroxyl)
C=O (amide)
Wavenumber [cm]-1


Figure 3

A:

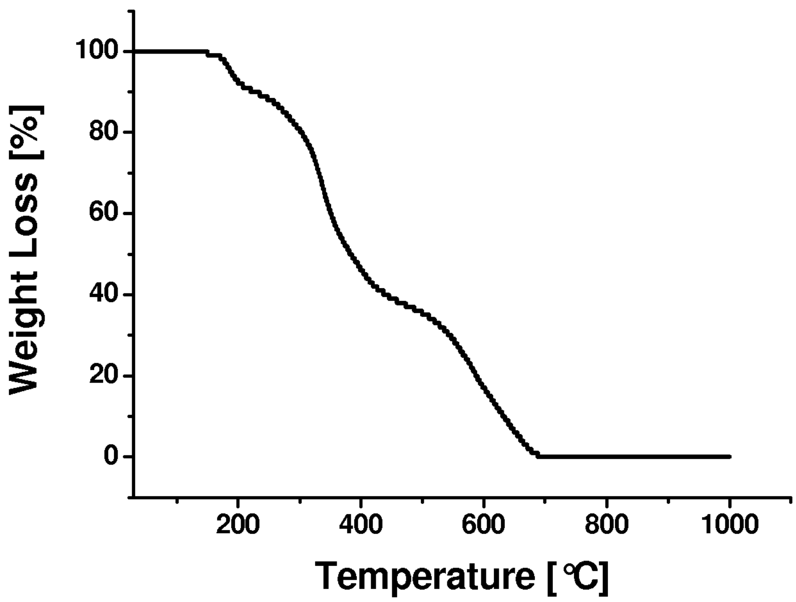
Weight Loss [%]
100
80
60
40
20
0
200
400
600
800
1000
Temperature [℃]

B:

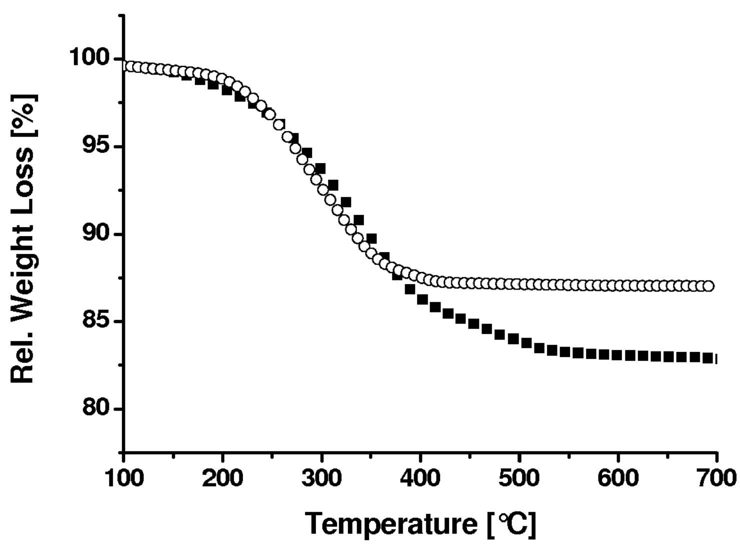
Rel. Weight Loss [%]
100
95
90
85
80
100
200
300
400
500
600
700
Temperature [℃]

Figure 4

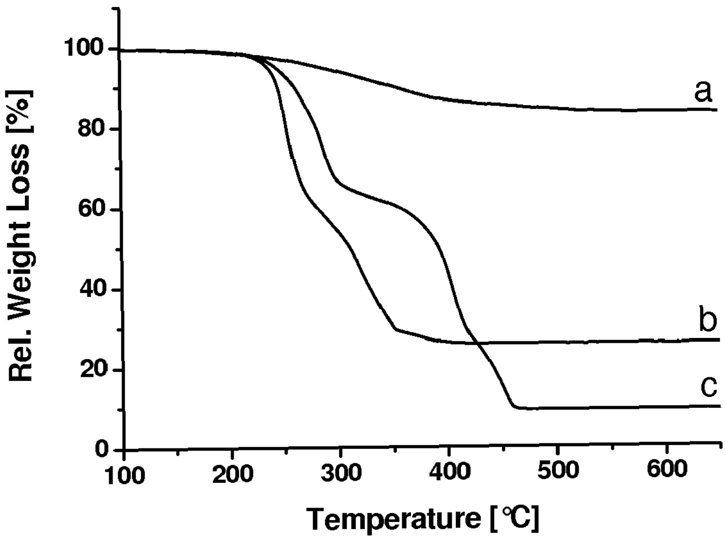
100
80
60
40
20
0
Rel. Weight Loss [%]
a
b
c
100
200
300
400
500
600
Temperature [°C]

Figure 5

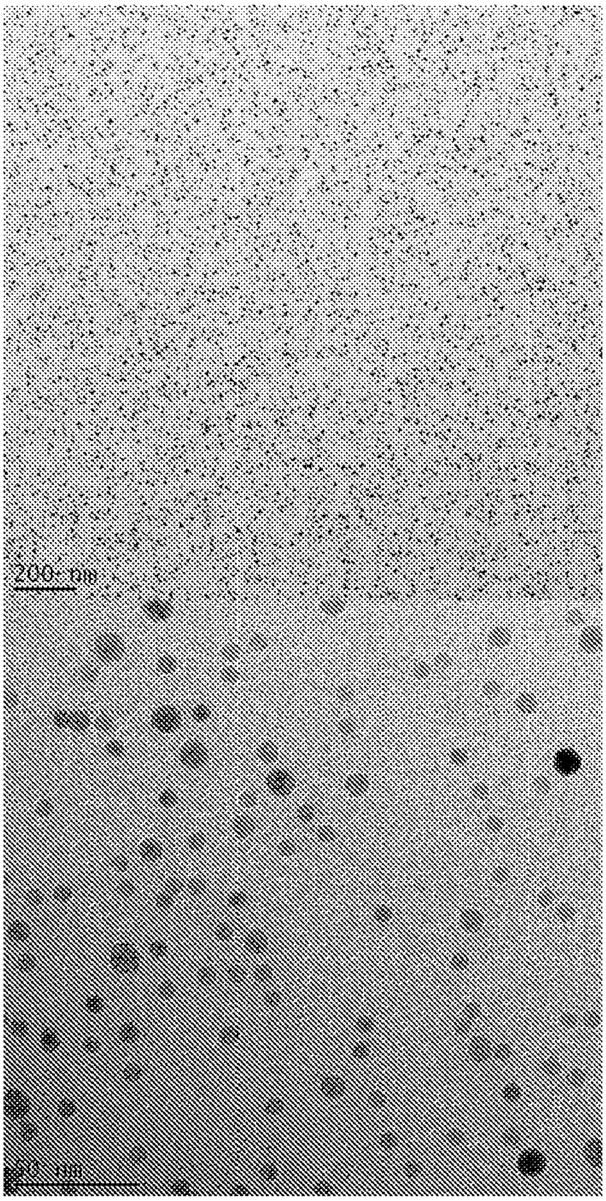


Figure 6

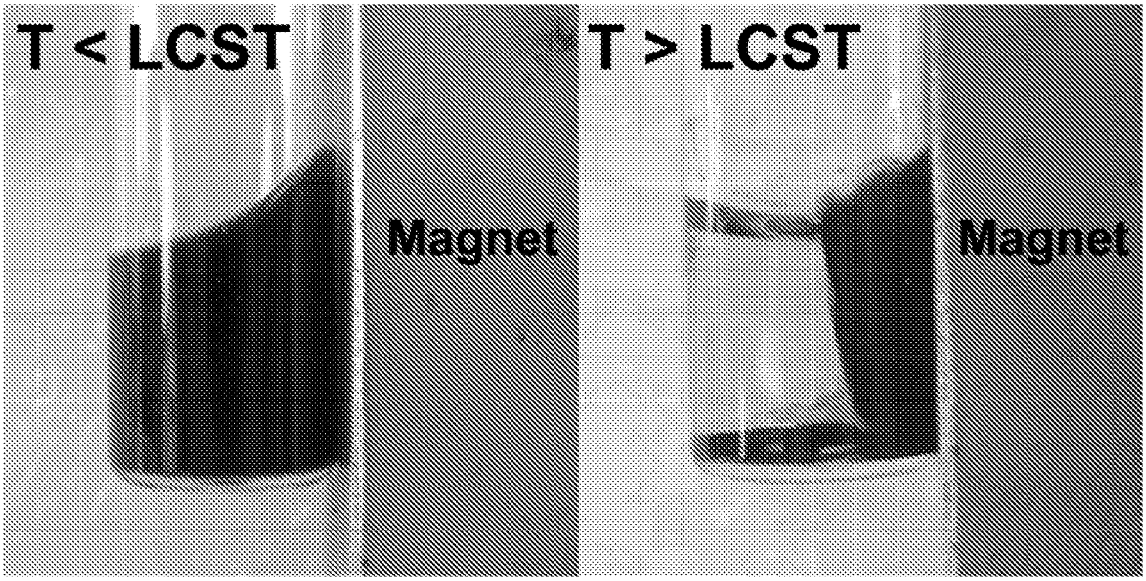


Figure 7

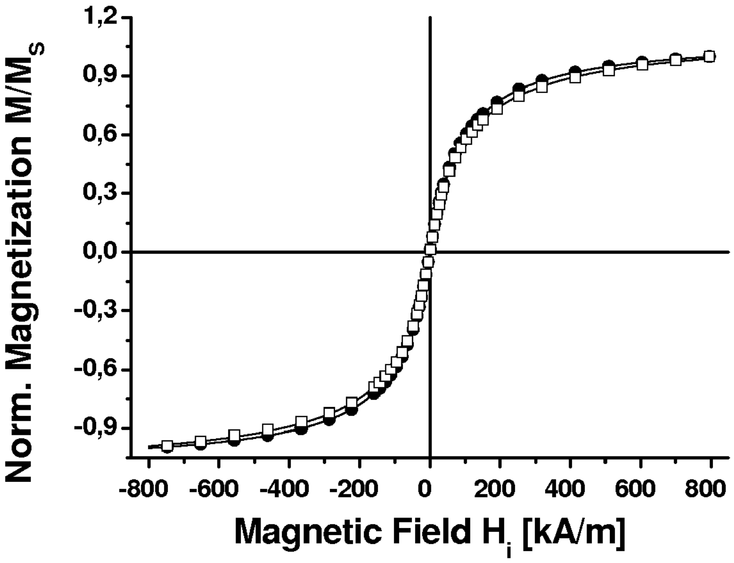

1,2
0,9
0,6
0,3
0,0
-0,3
-0,6
-0,9
Norm. Magnetization M/M$_S$
-800
-600
-400
-200
0
200
400
600
800
Magnetic Field H$_i$ [kA/m]


Figure 8

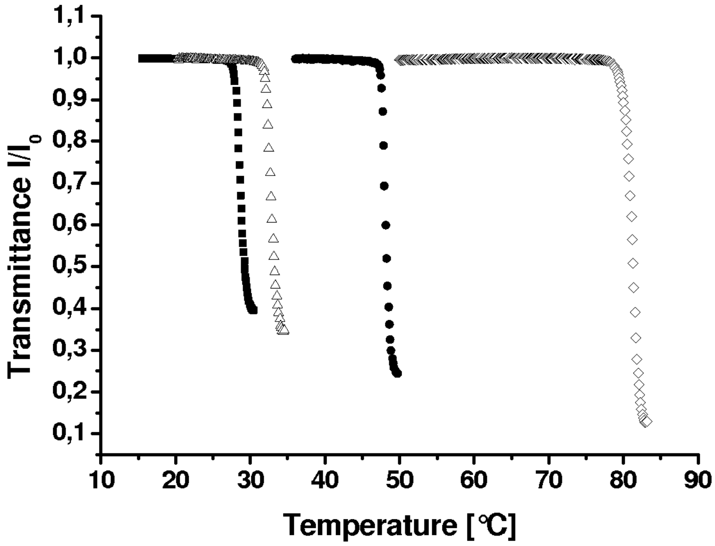
Transmittance I/I0
1,1
1,0
0,9
0,8
0,7
0,6
0,5
0,4
0,3
0,2
0,1
10 20 30 40 50 60 70 80 90
Temperature [°C]

**B:**

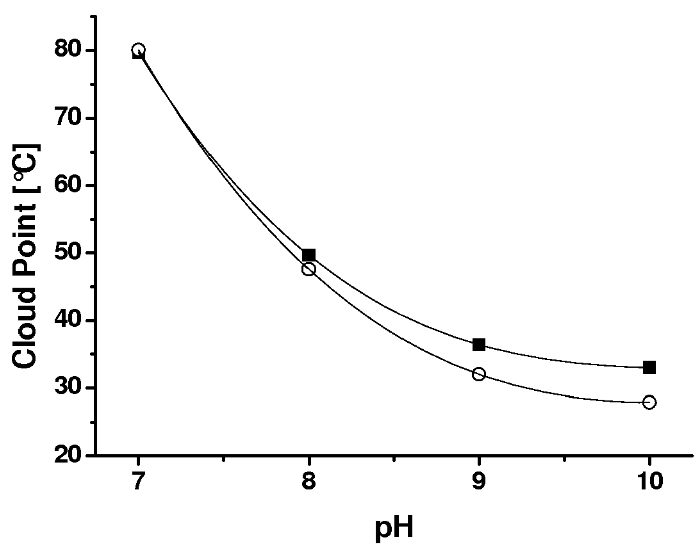
Cloud Point [°C]
80
70
60
50
40
30
20
7 8 9 10
pH

**Figure 9**

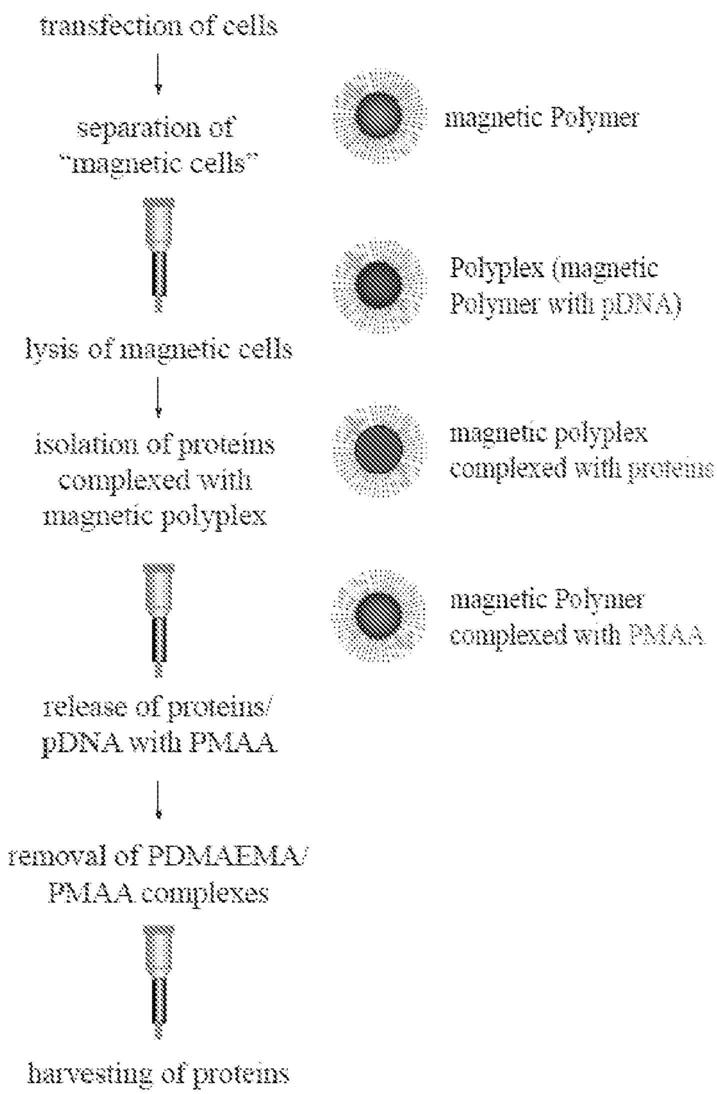
transfection of cells
separation of
"magnetic cells"
magnetic Polymer
Polyplex (magnetic
Polymer with pDNA)
lysis of magnetic cells
isolation of proteins
complexed with
magnetic polyplex
magnetic polyplex
complexed with proteins
magnetic Polymer
complexed with PMAA
release of proteins/
pDNA with PMAA
removal of PDMAEMA/
PMAA complexes
harvesting of proteins

**Figure 10**

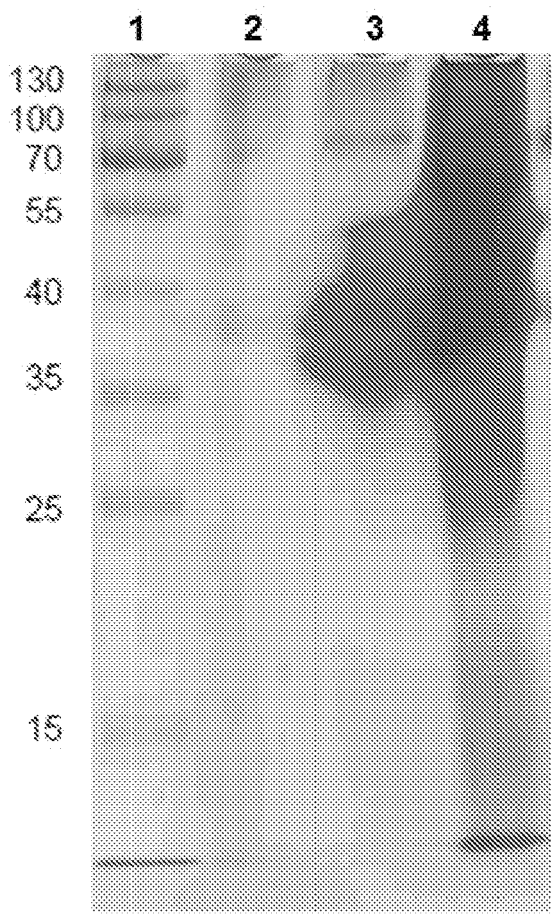


Figure 11

Europäisches Patentamt
European Patent Office
Office européen des brevets

# EUROPEAN SEARCH REPORT

Application Number
EP 11 16 6113

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2009/215166 A1 (GOEPFERICH ACHIM [DE] ET AL) 27 August 2009 (2009-08-27)<br>* paragraph [0082] - paragraph [0084] *<br>----- | 1-15 | INV.<br>G01N33/543<br>A61K48/00<br>C12N15/87 |
| A | WO 2007/133812 A2 (PHILADELPHIA HEALTH & EDUCATIO [US]; LUTZ GORDON JOHN [US]; WHEATLEY M) 22 November 2007 (2007-11-22)<br>* claims *<br>----- | 1-15 | |
| A | US 2005/090008 A1 (SEGURA TATIANA [CH] ET AL SEGURA TATIANA [US] ET AL)<br>28 April 2005 (2005-04-28)<br>* claims; figure 1 *<br>----- | 1-15 | |
| A | WO 02/00870 A2 (PLANK CHRISTIAN [DE]; BERGEMANN CHRISTIAN [DE])<br>3 January 2002 (2002-01-03)<br>* claims *<br>* page 41, paragraph 2 - page 42, paragraph 1; figure 27 *<br>----- | 1-15 | |

TECHNICAL FIELDS SEARCHED (IPC)

G01N
A61K
C12N

1

The present search report has been drawn up for all claims

| Place of search | Date of completion of the search | Examiner |
|---|---|---|
| The Hague | 14 September 2011 | Routledge, Brian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 16 6113

This annex lists the patent family members relating to the patent documents cited in the above-mentioned European search report. The members are as contained in the European Patent Office EDP file on
The European Patent Office is in no way liable for these particulars which are merely given for the purpose of information.

14-09-2011

| Patent document cited in search report | Publication date | Patent family member(s) | Publication date |
|---|---|---|---|
| US 2009215166 A1 | 27-08-2009 | DE 102005039154 A1 | 01-03-2007 |
| | | EP 1915413 A1 | 30-04-2008 |
| | | WO 2007020060 A1 | 22-02-2007 |
| WO 2007133812 A2 | 22-11-2007 | US 2009011004 A1 | 08-01-2009 |
| US 2005090008 A1 | 28-04-2005 | US 6890556 B1 | 10-05-2005 |
| WO 0200870 A2 | 03-01-2002 | EP 1297169 A2 | 02-04-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5525711 A
- US 4711955 A
- EP 302175 A
- US 4699880 A

### Non-patent literature cited in the description


- **E. MARSHALL.** *Science,* 1999, vol. 286, 2244-2245
- **S. HACEIN-BEY-ABINA.** *Science,* 2003, vol. 302, 415-419
- **D.W. PACK.** *Nat. Rev. Drug Discov.,* 2005, vol. 4, 581-593
- **A AKINC et al.** *J Gene Med,* 2005, vol. 7, 657-663
- **T. BIEBER et al.** *Journal of Controlled Release,* 2002, vol. 82, 441-454
- **K. LUBY-PHELPS.** *Proc. Natl Acad. Sci. USA,* 1987, vol. 84, 4910-4913
- **K. AOI ; M. OKADA.** *Prog. Polym. Sci,* 1996, vol. 21, 151-208
- **H. M. L. LAMBERMONT-THIJS et al.** *Polym. Chem.,* 2011, vol. 2, 313-322
- **M. LABET ; W. THIELEMANS.** *Chem. Soc. Rev.,* 2009, vol. 38, 3484-3504
- **G. RIESS.** *Prog. Polym. Sci.,* 2003, vol. 28, 1107-1170
- **FJ XU ; WT YANG.** *Prog. Polym. Sci,* 2011
- Macromolecular Engineering. Wiley-VCH, 2007, vol. 4
- **LV VINOGRADOVA.** *Russian Journal of Applied Chemistry,* 2010, vol. 83 (3), 351-378
- **H GAO et al.** *Progress in Polymer Science,* 2009, vol. 34, 317-350
- **S. BEHRENS.** *Nanoscale,* 2011, vol. 3, 877
- Macromolecular Engineering. Wiley-VCH, 2007
- **T HYEON et al.** *J. Am. Chem. Soc.,* 2001, vol. 123, 12798-12801
- **X. FAN et al.** *J. Am. Chem. Soc.,* 2005, vol. 127, 15843-15847
- **KAI SIMONS ; J. VICTOR SMALL ; TONY HUNTER ; JULIO E. CELIS ; NIGEL CARTER.** Cell Biology. A Laboratory Handbook. Elsevier Ltd, 2005, vol. 4
- **ZHIYU ZHANG et al.** *JALA,* 2007, vol. 12, 143-51
- **H. MORI et al.** *Journal of the American Chemical Society,* 2003, vol. 125, 3712-3713
- **H. MORI et al.** *Macromolecules,* 2004, vol. 37, 5228-5238
- **S. MUTHUKRISHNAN et al.** *Macromolecules,* 2005, vol. 38, 10631-10642
- **F. PLAMPER et al.** *Macromolecules,* 2007, vol. 40, 5689
- **FA PLAMPER et al.** *Macromolecules,* 2007, vol. 40, 5689-5697
- **AO IVANOV et al.** *Phys. Rev. E: Stat., Nonlinear, Soft Matter Phys.,* 2001, vol. 64, 041405
- **FA PLAMPER et al.** *Macromolecules,* 2007, vol. 40, 8361-8366